# EUROPEAN PATENT APPLICATION

(11) **EP 4 817 648 A1**
(43) Date of publication of application: **07.10.2026**
(21) Application number: 25770941.0
(22) Date of filing: 27.01.2025
(51) Int. Cl.: A61M 1/14, G16H 20/40

(54) **BLOOD PURIFICATION DEVICE, BLOOD PURIFICATION SYSTEM, AND INFORMATION TRANSFER METHOD**

(30) Priority: 28.03.2024 JP 2024053498
(71) Applicant: Nikkiso Co., Ltd., Tokyo 150-6022 (JP)
(72) Inventor: MACHIDA Yoshihiro, Higashimurayama-shi, Tokyo 189-0022 (JP)
(74) Representative: von Hirschhausen, Helge
(86) International application number: PCT/JP2025/002350
(87) International publication number: WO 2025/204059

(57) **Abstract**

There is provided a blood purification device that purifies blood of a patient, the blood purification device including: a storage unit that stores patient information including patient-specific data and treatment data; an output unit that outputs the patient information to the outside via an information acquisition device used by an operator of the blood purification device; a communication unit that transmits the patient information to the outside using a communication line; and a control unit that performs transfer of the patient information by controlling either the output unit or the communication unit.

## Description

### Technical Field

The present disclosure relates to a blood purification device that purifies blood of a patient, a blood purification system, and an information transfer method of transferring patient information, and particularly relates to a technique for transferring patient information from a blood purification device to another blood purification device.

### Background Art

In the related art, as an example of treatment by blood purification, dialysis treatment using a blood purification device including a dialyzer and a blood circuit is known. In the dialysis treatment, while extracorporeally circulating blood taken out from a patient by the blood circuit, blood purification is performed using the dialyzer connected to the blood circuit, and the purified blood is returned to the patient's body. In a medical facility such as a hospital, a plurality of blood purification devices are installed in a dialysis room, and it is possible to perform dialysis treatment on a plurality of patients under different conditions.

When the dialysis treatment is performed, individual treatment conditions determined for each patient are preset in the blood purification device. The presetting is performed by an operator (a medical worker such as a doctor or a nurse) of the blood purification device, or is performed by transmitting the treatment conditions from a server in the medical facility to each blood purification device. For example, Patent Literature 1 discloses a technique in which, in a case where a blood purification device to be used fails, pieces of identification information of patients and the other blood purification devices are transmitted from the other blood purification devices to the server and the treatment conditions calculated by the server are returned to the other blood purification devices.

In addition, treatment conditions and various types of information which are set in the blood purification device are stored in the server, and various analyses may be performed. For example, Patent Literature 2 discloses a technique in which an image displayed on a display of a blood purification device is acquired by an image acquiring device and the image is transmitted from the image acquiring device to a server. Further, the server decodes treatment conditions and various types of information from the received image.

On the other hand, there is a case where the dialysis treatment is interrupted due to a failure of the blood purification device or another reason during the dialysis treatment, and as a result, the treatment is continued by another blood purification device or the dialysis treatment is stopped. For continuation of treatment by another blood purification device, it is important that patient movement and rapid transfer of various types of information are possible from the blood purification device (initial device) which cannot be used to a blood purification device (alternative device) which is newly selected and that a lead time until the treatment is restarted is short. This is because, when it takes time to restart the treatment, the patient's blood stagnates in the blood circuit and a risk of blood coagulation increases.

### Citation List

### Patent Literature

Patent Literature 1: JP 2015-181726 A
Patent Literature 2: JP 2014-517709 A

### Summary of Invention

### Technical Problem

However, although a preferred method for transferring various types of information varies depending on a state of the blood purification device or an installation environment of the blood purification device, it is not possible to sufficiently cope with these problems, and it is difficult to shorten the lead time until the treatment is restarted.

For example, in the blood purification system disclosed in Patent Literature 1, although it is possible to transmit the treatment conditions from the server to the alternative device, progress information of the treatment cannot be extracted from the initial device, and the transfer of various types of information to the alternative device becomes insufficient. In addition, in the blood purification system disclosed in Patent Literature 1, since pieces of identification information of the patient and the alternative device are input on the alternative device side, a human error such as an input error may occur, and it takes time to input the pieces of identification information. Further, the method disclosed in Patent Literature 2 that is for transferring information is limited to a form in which images related to various types of information and an image acquiring device are used. As a result, depending on the state of the blood purification device or the installation environment of the blood purification device, transfer of the information may not be possible, or it may take time to transfer the information.

The present disclosure has been made in view of such problems, and an object of the present disclosure is to provide a blood purification device, a blood purification system, and an information transfer method capable of easily and accurately transferring information and shortening a lead time until treatment is restarted.

### Solution to Problem

According to one aspect of the present disclosure, there is provided "a blood purification device that purifies blood of a patient, the blood purification device including: a storage unit that stores patient information including patient-specific data and treatment data; an output unit that outputs the patient information to the outside via an information acquisition device used by an operator of the blood purification device; a communication unit that transmits the patient information to the outside using a communication line; and a control unit that performs transfer of the patient information by controlling either the output unit or the communication unit."

According to another aspect of the present disclosure, there is provided "a blood purification system including: a first blood purification device that purifies blood of a patient; a second blood purification device that is selected as an alternative device for the first blood purification device; and a server that is connected to the first blood purification device and the second blood purification device via a communication line, in which the first blood purification device includes a storage unit that stores patient information including patient-specific data and treatment data, an output unit that outputs the patient information to the outside via an information acquisition device used by an operator of the first blood purification device, a communication unit that transmits the patient information to the server using the communication line, and a control unit that performs processing for transferring the patient information to the second blood purification device by controlling either the output unit or the communication unit."

According to still another aspect of the present disclosure, there is provided "an information transfer method of transferring patient information from a blood purification device that purifies blood of a patient, the information transfer method including: a step of storing the patient information including patient-specific data and treatment data in a storage unit of the blood purification device; a step of selecting either an operator-intervention-type transfer method or a communication-type transfer method, the operator-intervention-type transfer method being a method of transferring information by outputting the patient information to the outside via an information acquisition device used by an operator of the blood purification device, and the communication-type transfer method being a method of transferring information by transmitting the patient information to the outside using a communication line; and a step of transferring the patient information based on the selected transfer method."

### Advantageous Effects of Invention

According to the present disclosure, it is possible to provide a blood purification device, a blood purification system, and an information transfer method capable of easily and accurately transferring information and shortening a lead time until treatment is restarted.

Note that the above effect is merely an example for convenience of description, and the effect according to the present disclosure is not limited to the above. In addition to the above effects, according to the present disclosure, any effect described in the present disclosure can be exhibited.

### Brief Description of Drawings

[Fig. 1] Fig. 1 is a schematic diagram illustrating a configuration of a blood purification system according to a first embodiment.
[Fig. 2] Fig. 2 is a schematic diagram illustrating an example of a use state of the blood purification device according to the first embodiment.
[Fig. 3] Fig. 3 is a block diagram illustrating an electrical configuration of the blood purification device according to the first embodiment.
[Fig. 4] Fig. 4 is a functional block diagram related to information transfer of the blood purification device according to the first embodiment.
[Fig. 5] Fig. 5 is a functional block diagram related to information transfer of the blood purification device according to the first embodiment.
[Fig. 6] Fig. 6 is a block diagram illustrating an electrical configuration of an in-hospital server according to the first embodiment.
[Fig. 7] Fig. 7 is a block diagram illustrating an electrical configuration of a mobile terminal according to the first embodiment.
[Fig. 8] Fig. 8 is a block diagram illustrating an electrical configuration of a cloud server according to the first embodiment.
[Fig. 9A] Fig. 9A is a sequence diagram related to information transfer in the blood purification system according to the first embodiment.
[Fig. 9B] Fig. 9B is an example of a screen displayed in the blood purification device according to the first embodiment.
[Fig. 9C] Fig. 9C is an example of a screen displayed in the blood purification device according to the first embodiment.
[Fig. 9D] Fig. 9D is an example of an identification marker displayed in the blood purification device according to the first embodiment.
[Fig. 9E] Fig. 9E is an example of patient information displayed in the blood purification device according to the first embodiment.
[Fig. 9F] Fig. 9F is an example of transfer procedure information displayed in the blood purification device according to the first embodiment.
[Fig. 9G] Fig. 9G is an example of transfer procedure information displayed in the blood purification device according to the first embodiment.
[Fig. 9H] Fig. 9H is an example of difference information displayed in the blood purification device according to the first embodiment.
[Fig. 10] Fig. 10 is a sequence diagram related to information transfer in the blood purification system according to the first embodiment.
[Fig. 11] Fig. 11 is a sequence diagram related to information transfer in the blood purification system according to the first embodiment.
[Fig. 12] Fig. 12 is a sequence diagram related to information transfer in the blood purification system according to the first embodiment.
[Fig. 13] Fig. 13 is a functional block diagram related to information transfer of a blood purification device according to a second embodiment.
[Fig. 14] Fig. 14 is a sequence diagram related to information transfer in the blood purification system according to the second embodiment.
[Fig. 15] Fig. 15 is a flowchart related to selection of a transfer method in the blood purification device according to the second embodiment.
[Fig. 16] Fig. 16 is a functional block diagram related to information transfer of a blood purification device according to a third embodiment.
[Fig. 17] Fig. 17 is a sequence diagram related to information transfer in the blood purification system according to the third embodiment.
[Fig. 18] Fig. 18 is a functional block diagram related to information transfer of a blood purification device according to a fourth embodiment.
[Fig. 19] Fig. 19 is a sequence diagram related to information transfer in a blood purification system according to the fourth embodiment.

### Description of Embodiments

Hereinafter, a blood purification device according to the present disclosure and a blood purification system including the blood purification device will be described in detail with reference to the drawings. Note that the present disclosure is not limited to the content to be described below, and can be arbitrarily changed and implemented without changing the gist of the present disclosure. In addition, the drawings used in each embodiment schematically illustrate the blood purification device according to the present disclosure and the blood purification system including the blood purification device, and partial emphasis, enlargement, reduction, omission, or the like is performed in order to enhance understanding. The drawings may not accurately represent a scale, a shape, and the like of each component. Further, some numerical values used in each embodiment are merely examples, and can be variously changed as necessary. In addition, the same reference numerals are given to the same configurations in the drawings.

### <First Embodiment>

### (Configuration of Blood Purification System)

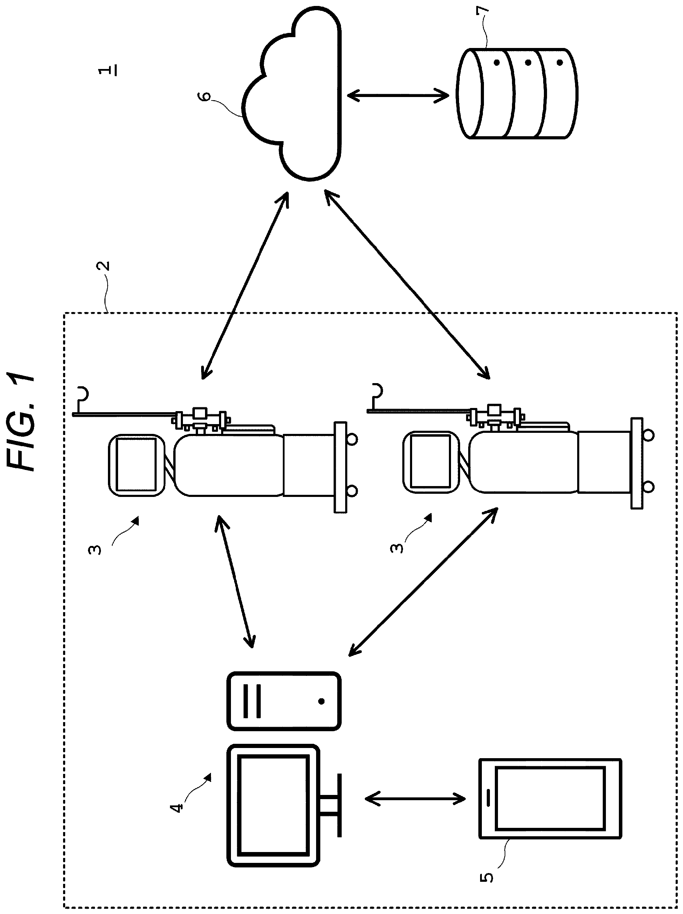

First, a configuration of a blood purification system including a blood purification device according to the present disclosure will be described with reference to Fig. 1. Fig. 1 is a schematic diagram illustrating a configuration of a blood purification system 1 according to the present embodiment. In particular, Fig. 1 illustrates a communication relationship between constituent devices included in the blood purification system 1.

As illustrated in Fig. 1, the blood purification system 1 according to the present embodiment includes a plurality of blood purification devices 3 installed in a hospital 2 which is an example of a treatment facility, an in-hospital server (in-facility server) 4, and a mobile terminal 5. As illustrated in Fig. 1, the blood purification system 1 also includes a cloud server (out-of-facility server) 7 communicably connected to the blood purification devices 3 in the hospital via the network 6. Note that the treatment facility is not limited to a hospital, and may be another facility as long as treatment related to blood purification is possible. Further, the network 6 may be configured by a wireless network, a wired network, or a combination of a wireless network and a wired network.

In the hospital 2, the blood purification device 3, the in-hospital server 4, and the mobile terminal 5 are communicably connected to each other via an in-hospital network (a wireless network, a wired network, or a combination of a wireless network and a wired network). Note that, in Fig. 1, the mobile terminal 5 is connected only to the in-hospital server 4, but is also communicably connected to the blood purification device 3. In addition, the in-hospital server 4 and the mobile terminal 5 provided in the hospital 2 may be communicably connected to the cloud server 7 via the network 6. In a case where the in-hospital server 4 has a function as a communication server, the blood purification device 3 and the mobile terminal 5 may perform communication with the cloud server 7 outside the hospital via the in-hospital server 4.

In the blood purification system 1, each of the blood purification devices 3 is a device that performs treatment related to blood purification of a patient, and a specific configuration of the blood purification device 3 will be described later. Each of the blood purification devices 3 can transmit various types of data and information acquired in the device to the in-hospital server 4 or the cloud server 7. Here, the data is basically assumed to include a numerical value, a symbol, a character, or the like obtained by processing a signal or the like. In addition, the information is basically assumed to be obtained by collecting or processing the data, and is assumed to include, for example, content that can be used by a recipient as a material for further consideration or can be used by a recipient. However, the data and the information may be used in accordance with the content and the preceding and following contexts without matching with the above assumption. Note that, although only two blood purification devices 3 are illustrated in Fig. 1, the number of blood purification devices 3 is not limited thereto, and the blood purification system 1 may include three or more blood purification devices 3. In addition, the blood purification devices 3 are not limited to devices having the same type, and may be devices having different types and versions.

The in-hospital server 4 is a computer that manages various types of data and information in the hospital 2 and processes these pieces of data and information, and a specific configuration of the in-hospital server 4 will be described later. For example, the in-hospital server 4 may process data or information received from the blood purification device 3, and transmit a processing result to the blood purification device 3 or the mobile terminal 5. Note that the in-hospital server 4 may be not only a device physically installed inside the hospital 2 but also a device that functions as a server of the hospital 2 while being installed outside the hospital 2. In addition, in Fig. 1, the in-hospital server 4 is described as a single server, but various configurations and processes of the in-hospital server 4 to be described later can be distributed to a plurality of other server devices. In this case, in a case where the functions of the in-hospital server 4 are distributed, a part may be provided inside the hospital 2 and the remaining part may be provided outside the hospital.

The mobile terminal 5 is a device that processes the various types of data which are received and transmits the data to the blood purification device 3 or the in-hospital server 4, and a specific configuration of the mobile terminal 5 will be described later. For example, the mobile terminal 5 may be a device that can perform wireless communication and is represented by a smartphone, but is not limited thereto. For example, the mobile terminal 5 may be a feature phone, a portable information terminal, a PDA, a laptop personal computer, a desktop personal computer, or the like. Note that, although only one mobile terminal 5 is illustrated in Fig. 1, the number of the mobile terminals 5 is not limited thereto, and the blood purification system 1 may include two or more mobile terminals 5.

Similarly to the in-hospital server 4, the cloud server 7 is a computer that manages various types of data and information in the hospital 2 and processes these pieces of data and information, and a specific configuration of the cloud server 7 will be described later. For example, the cloud server 7 may process the data or the information received from the blood purification device 3, and transmit a processing result to the blood purification device 3. Note that, in Fig. 1, the cloud server 7 is described as a single server, but various configurations and processes of the cloud server 7 to be described later can be distributed to a plurality of other server devices.

Note that the blood purification system 1 only needs to be able to transfer patient information related to a patient who receives blood purification treatment from one blood purification device 3 (an initial device 3a to be described later) to another blood purification device (an alternative device 3b to be described later), and may not include all of the in-hospital server 4, the mobile terminal 5, and the cloud server 7. That is, in an environment in which external communication is not possible, the blood purification system 1 may not include the cloud server 7 because connection to the cloud server 7 cannot be performed. In addition, in a case where information management of the hospital 2 can be performed only by the cloud server 7 without providing the in-hospital server 4, the blood purification system 1 may not include the in-hospital server 4. Further, the blood purification system 1 may not include the mobile terminal 5, and the patient information may be transferred without using the mobile terminal 5.

Then, the patient information includes patient-specific data and treatment data. The specific data includes data for identifying a patient and various types of data related to the patient. For example, the specific data may include data related to treatment such as a name, a gender, a weight, a height, and a blood pressure of a patient. Further, the specific data may include an ID for identifying the patient, and the like. On the other hand, the treatment data may include data on treatment conditions, data on medical materials, data (specification information) on accessories attached to the blood purification device, data on a medication agent (anticoagulant), and treatment progress data of the patient. Specifically, the treatment conditions may include a treatment time, a treatment mode (type), and a prescription amount. The accessories may include a blood circuit, a blood purifier (dialyzer), a syringe, a saline solution, and dialysate. The treatment progress data is data related to past and currently ongoing treatments of the patient, and may include internal parameters, a temperature, a concentration, and a pressure in the blood purification device 3.

### (Configuration of Blood Purification Device)

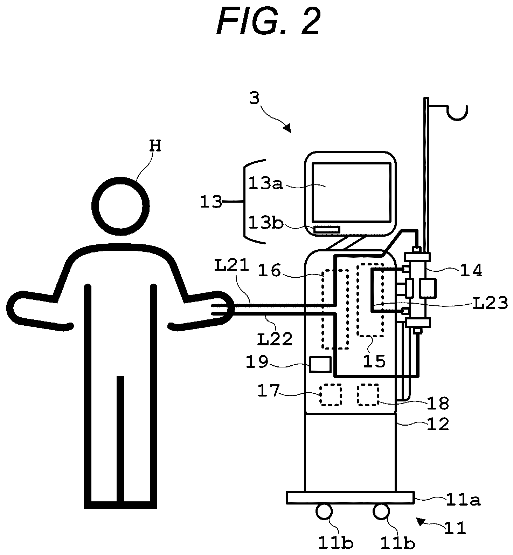

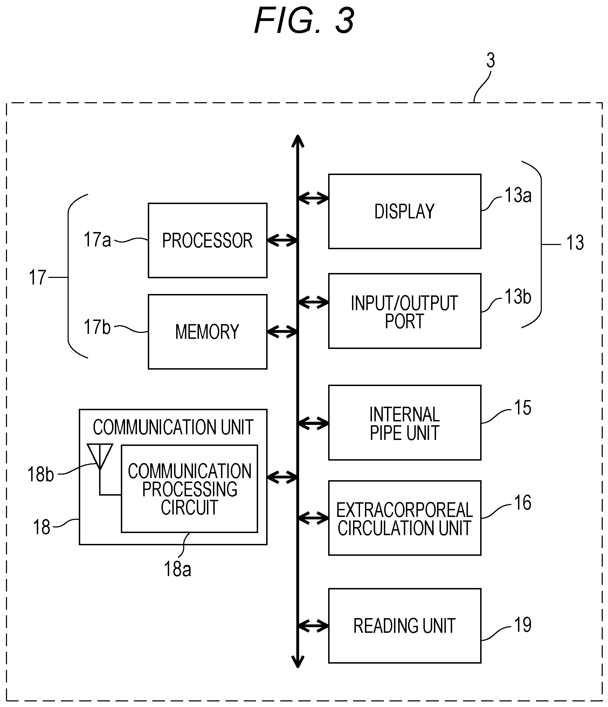

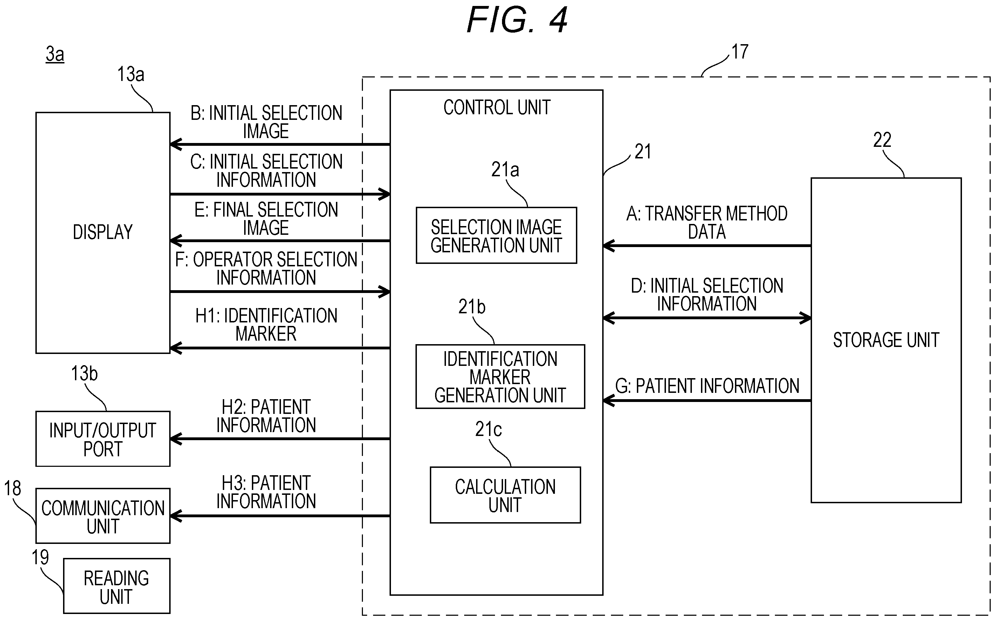

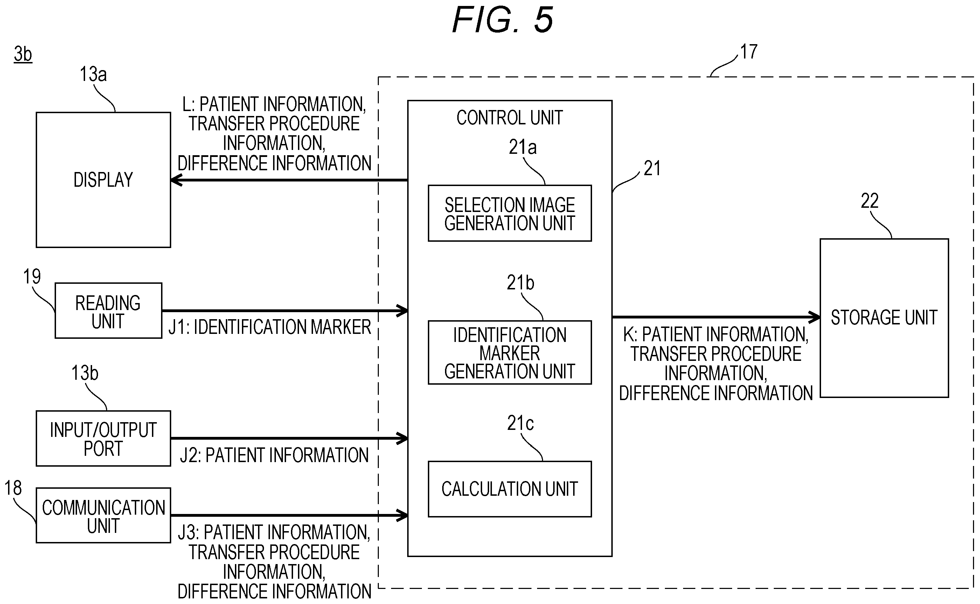

Next, a configuration of the blood purification device according to the present disclosure will be described with reference to Fig. 2 to Fig. 5. Fig. 2 is a schematic diagram illustrating an example of a use state of the blood purification device 3 according to the present embodiment. Fig. 3 is a block diagram illustrating an electrical configuration of the blood purification device 3 according to the present embodiment. Fig. 4 is a functional block diagram related to information transfer of the blood purification device 3 according to the present embodiment, and is particularly a functional block diagram as an initial device 3a that is an information transfer source device. Fig. 5 is a functional block diagram related to information transfer of the blood purification device 3 according to the present embodiment, and is particularly a functional block diagram as an alternative device 3b that is an information transfer destination device.

As illustrated in Fig. 2, the blood purification device 3 is configured with a dialysis device for performing dialysis treatment on a patient H. Specifically, the blood purification device 3 includes a main body 12 provided on a base unit 11, an input/output unit 13 connected to an upper portion of the main body 12, and a blood purifier 14 provided on a side of the main body 12. In addition, the main body 12 of the blood purification device 3 includes an internal pipe unit 15 for circulating dialysate with the blood purifier 14, an extracorporeal circulation unit 16 for circulating blood that is a body fluid of the patient H outside the body, and an information processing device 17. With such a configuration, the blood purification device 3 can take out the blood of the patient H outside the body, remove unnecessary or toxic substances or moisture from the blood in the blood purifier 14, and return the purified blood to the patient H.

The base unit 11 includes a plate-like base 11a connected to a bottom portion of the main body 12, and four casters 11b provided on the base 11a. Thereby, it is possible to easily move the blood purification device 3. Note that the number of the casters 11b is not limited to four, and may be three or five or more as long as the blood purification device 3 can be moved.

The main body 12 is configured with a housing having a substantially rectangular parallelepiped shape. In addition, various constituent devices and various constituent components included in the blood purification device 3 are disposed inside and on a surface of the main body 12. Here, examples of the constituent device include pumps such as a dual pump, a water removing pump, a degassing pump, a pressurizing pump, a stock solution pump, and a blood pump, and various detectors.

The input/output unit 13 includes a display 13a and an input/output port 13b for connecting an external storage device (not illustrated). Note that the display 13a and the input/output port 13b are not necessarily provided side by side, and the input/output port 13b may be provided on a front surface, a back surface, or the like of the main body 12. In addition, in the input/output port 13b, the input port and the output port may be separated.

Next, as illustrated in Fig. 3, in the blood purification device 3, the display 13a, the input/output port 13b, the internal pipe unit 15, the extracorporeal circulation unit 16, the information processing device 17, the communication unit 18, and a reading unit 19 are electrically connected to each other via a control line and a data line. Thereby, in the blood purification device 3, signals, data, and information between various electrical components can be transmitted and received, and various controls by the information processing device 17 can also be performed.

In the present embodiment, a case where a hemodialysis device is an example of the blood purification device 3 has been described, but the present disclosure is not limited thereto. For example, a device for acute blood purification, a peritoneal dialysis device, an ultrafiltration device, or a hemofiltration device can also be an example of the blood purification device 3. That is, in the present disclosure, any device may be used as long as the transfer processing of patient information from the blood purification device 3 can be performed.

### [Display]

Next, the display 13a includes a touch-panel-type input interface and a general screen-type output interface. That is, the display 13a in the present embodiment is a touch panel including an input/output interface, and has an input function and an output function. Here, the input detection method by the touch panel may be any method such as a capacitance type or a resistive film type.

Note that the input interface may be separated from the display 13a. In this case, a keyboard including physical key buttons such as a numeric keypad or character input keys, and an input device such as a mouse may be provided in the blood purification device 3.

### [Input/Output Port]

The input/output port 13b is configured with terminals of the interface used for input/output of information and data with various devices connected to the outside of the blood purification device 3. That is, the input/output port 13b can be connected to various external storage devices (not illustrated) that are a type of an information acquisition device, and has an input/output function that enables input/output of information and data between the blood purification device 3 and the external storage device. For example, the external storage device that can be connected to the input/output port 13b may be a Universal Serial Bus (USB) memory, a CompactFlash (CF) card, a near field communication (NFC) card, or the like.

### [Blood Purifier]

The blood purifier 14 has a blood inlet and a blood outlet at both ends of a housing as blood-side ports, and has a dialysate inlet and a dialysate outlet at a side of the housing as dialysate-side ports. An arterial blood circuit L21 is connected to the blood inlet, and a venous blood circuit L22 is connected to the blood outlet.

The blood purifier 14 accommodates a plurality of hollow fiber membranes (not illustrated) therein, and the hollow fibers constitute a blood purification membrane for purifying blood. In the blood purifier 14, a blood flow path through which the blood of the patient H flows and a dialysate flow path through which the dialysate flows are formed through the blood purification membrane. In addition, in the hollow fiber membranes included in the blood purification membrane, a large number of minute holes penetrating the outer peripheral surface and the inner peripheral surface are formed, and impurities and the like in blood can permeate into the dialysate through the hollow fiber membranes.

Note that the blood purifier 14 is not limited to the dialyzer having the above-described configuration. For example, the blood purifier 14 may be an adsorption-type blood purifier used for endotoxin adsorption treatment, activated carbon adsorption treatment, bilirubin adsorption treatment, or the like. In addition, the blood purifier 14 may also be a hemodiafilter.

### [Internal Pipe Unit]

The internal pipe unit 15 is provided inside the main body 12, and is connected to two terminals provided on the side surface of the blood purifier 14 via a pipe L23. For example, the internal pipe unit 15 includes various components such as a pipe, a pump, a valve, a sensor, and a filter. More specifically, the internal pipe unit 15 may include a dual pump, a water removing pump, a degassing pump, a pressurizing pump, a pressure reducing valve, an electromagnetic valve, a temperature sensor, a pressure sensor, a chemical liquid filter, and the like. In addition, a flexible material such as a vinyl chloride tube or a silicone tube may be used for the pipe.

The internal pipe unit 15 is assembled by appropriately selecting the components described above according to the pipe configuration and the type, and has a structure capable of circulating and washing the dialysate. In addition, the internal pipe unit 15 has a structure that enables introduction and discharge of the dialysate into and from the blood purifier 14. Note that, since these specific configurations are not characteristics of the blood purification device according to the present disclosure, the description thereof will be omitted.

### [Extracorporeal Circulation Unit]

The extracorporeal circulation unit 16 has a structure in which an arterial blood circuit L21 is connected to the blood introduction side with respect to the blood purifier 14, and a venous blood circuit L22 is connected to the blood discharge side with respect to the blood purifier 14. In addition, a liquid level adjustment circuit (not illustrated) is connected between the arterial blood circuit L21 and the venous blood circuit L22 so as to bypass the blood purifier 14. For example, the extracorporeal circulation unit 16 includes various components such as a circuit, a pump, a valve, a sensor, and a detector. More specifically, the extracorporeal circulation unit 16 may include connectors, an electromagnetic valve, a bubble detector, a blood pump, a blood concentration detector, an air trap chamber, and the like. In addition, for example, a flexible material such as a vinyl chloride tube or a silicone tube may be used for each circuit.

In a case where the blood purification device 3 is operated to purify the blood of the patient H, an arterial puncture needle and a venous puncture needle (both not illustrated) are connected to the connectors of the extracorporeal circulation unit, and each puncture needle is inserted into the arm of the patient H. Then, in the arterial blood circuit L21, the amount and the concentration of blood taken out from the patient H are detected, and in the venous blood circuit L22, the amount and the concentration of blood to be returned to the patient H are detected.

An operation of each of the electromagnetic valves, the pumps, and the detectors which are described above and are included in the internal pipe unit 15 and the extracorporeal circulation unit 16 is controlled based on a control signal supplied from the information processing device 17. In addition, the electromagnetic valve, the pump, and the detector can transmit, to the information processing device 17, state parameters indicating operation states of the respective constituent devices or the respective constituent components, or the blood purification device 3 including the components. For example, these electromagnetic valves, pumps, and detectors may transmit state parameters measured by various built-in sensors to the information processing device 17. That is, the state parameters may be measurement values measured by various sensors. Note that, in a case where various sensors are not built-in, the state parameters may be measured and transmitted by various sensors disposed in the vicinity of each constituent device or the constituent component.

Similarly, each of the above-described pressure sensors can also transmit a measurement value to the information processing device 17. Thereby, the information processing device 17 can acquire various state parameters indicating the operation state of the extracorporeal circulation unit 16 of the blood purification device 3. On the other hand, since the connector, the filter, and the air trap chamber described above do not have a sensing function, the use states of the connector, the filter, and the air trap chamber may be recognized by a temperature sensor or a pressure sensor provided in the vicinity of the components.

### [Communication Unit]

Next, as illustrated in Fig. 3, the communication unit 18 includes a communication processing circuit 18a and an antenna 18b. The communication unit 18 transmits and receives information to and from the in-hospital server 4, the mobile terminal 5, and the cloud server 7 installed separately from the blood purification device 3 via the communication processing circuit 18a and the antenna 18b.

The communication processing circuit 18a may execute processing based on a broadband wireless communication system represented by an LTE system. In addition, the communication processing circuit 18a may execute processing based on a system related to narrowband wireless communication such as wireless LAN represented by IEEE 802.11 or Bluetooth (registered trademark). Further, the communication processing circuit 18a may execute processing based on a system related to non-contact wireless communication. Then, the communication processing circuit 18a may use wired communication instead of or in addition to such wireless communication.

### [Reading Unit]

The reading unit 19 is an example of an information acquisition device, and is a general reader device for a one-dimensional or two-dimensional identification marker. In the present embodiment, the reading unit 19 reads an identification marker displayed on the display 13a of another blood purification device 3 (that is, the initial device 3a) or the display of the mobile terminal 5. For example, the reading unit 19 optically detects the identification marker, and converts the identification marker into specific information (patient information) by analyzing patterns of a black portion and a white portion (a blank portion).

Specifically, the reading unit 19 may include a light source and an optical sensor, and may also have a structure for scanning the identification marker. In this case, the output of the optical sensor is digitized while being amplified from a weak analog signal. In particular, since reflectance of the blank portion is high, the blank portion is converted into a digital signal of "0", and since reflectance of the black portion is low, the black portion is converted into a digital signal of "1". Then, the pattern is decoded by the information processing device 17, and information can be output by various interfaces.

Note that the reading unit 19 may be connected to the main body 12 by an extendable cable. Thereby, the reading unit 19 can be separated from the main body 12, and the reading unit 19 can be brought close to the identification marker to be read. In addition, in a case where the blood purification device 3 does not read an identification marker, the reading unit 19 may not be provided.

### [Information Processing Device]

As illustrated in Fig. 3, the information processing device 17 according to the present embodiment includes a processor 17a and a memory 17b.

The processor 17a is configured with a graphics processing unit (GPU) or a central processing unit (CPU), and functions as a control unit that controls other connected constituent devices or constituent components based on various programs stored in the memory 17b. Specifically, the processor 17a reads a program for executing blood purification processing or a program for executing OS from the memory 17b and executes the program. In addition, the processor 17a executes processing related to information transfer and initial setting in the blood purification device 3. Note that the processor 17a may be configured with a single GPU or CPU, but may be configured with a combination of a plurality of GPUs or CPUs.

The memory 17b includes a ROM, a RAM, a nonvolatile memory, an HDD, and the like, and functions as a storage unit. The ROM stores instruction commands for executing the blood purification processing as a program. In addition, the ROM stores, as a program, instruction commands for executing processing related to information transfer and initial setting in the blood purification device 3. Further, the RAM is used to write and read data while the program stored in the ROM is being processed by the processor 17a. Then, the nonvolatile memory is a storage device in which writing and reading of data are executed by execution of the program, and the data written in the nonvolatile memory is stored even after the execution of the program is completed.

As described above, in the present embodiment, the program of the processing related to initial setting of the information transfer processing in the blood purification device 3 and transfer method data indicating the content of the transfer method are stored, and particularly, the program related to four types of transfer methods and transfer method data indicating the content of the transfer method are stored in the memory 17b. Specifically, as a first transfer method, a method of generating an identification marker related to patient information, displaying the identification marker on the display 13a, and executing information transfer by reading of a reading device such as the mobile terminal 5 is stored. In addition, as a second transfer method, a method of storing patient information in an external storage device via the input/output port 13b and executing information transfer is stored. Further, as a third transfer method, a method of transmitting patient information to the in-hospital server 4 via the communication unit 18 and executing information transfer is stored. Then, as a fourth transfer method, a method of transmitting patient information to the cloud server 7 via the communication unit 18 and executing information transfer is stored. Here, the first and second transfer methods are methods in which an operator of the blood purification device 3 needs to intervene (operator-intervention-type transfer method), and the third and fourth transfer methods are methods that can be automatically executed without the intervention of the operator (communication-type transfer method). Note that processing executed by the program will be described later.

In the first transfer method, information transfer is performed by converting patient information into an identification marker and reading the identification marker, and thus, information security can be ensured. In addition, in the second transfer method, information transfer is performed by inserting and removing an external storage device, and thus, information transfer can be easily performed as compared with the first transfer method. On the other hand, in the third transfer method and the fourth transfer method, information transfer is automatically performed using the communication line, and thus, it is not necessary for the operator to intervene. Thereby, a burden related to the information transfer is reduced.

In addition, in the memory 17b, at least one of the first to fourth transfer methods initially selected by the operator is stored as initial selection information. That is, the memory 17b stores a method selected as a candidate of the transfer method in a case where information transfer of the patient information is required. Note that a flow related to initial selection and storing of initial selection information will be described later.

Further, in the memory 17b, patient information related to patient treatment is stored as the latest information. Then, the memory 17b also stores transfer procedure information, which is a procedure related to transfer of the patient information, and difference information between new patient information and past patient information. The content of these pieces of information will be described later.

### (Functional Configuration of Information Processing Device)

Next, a functional configuration of the blood purification device 3 according to the present embodiment will be described with reference to Fig. 4 and Fig. 5. Here, Fig. 4 and Fig. 5 are functional block diagrams related to information transfer of the blood purification device 3 according to the first embodiment. In particular, Fig. 4 illustrates a functional configuration in a case where the blood purification device 3 is an information transfer source (that is, in the case of the initial device 3a), and Fig. 5 illustrates a functional configuration in a case where the blood purification device 3 is an information transfer destination (that is, in the case of the alternative device 3b). In addition, Fig. 4 and Fig. 5 also illustrate flows of information and data between the respective components.

As illustrated in Fig. 4 and Fig. 5, the information processing device 17 includes a control unit 21 and a storage unit 22. In addition, the control unit 21 includes a selection image generation unit 21a, an identification marker generation unit 21b, and a calculation unit 21c. These units are implemented by causing the processor 17a and the memory 17b of the information processing device 17 to independently function, or are implemented by causing the processor 17a to read a program stored in the memory 17b and execute the program.

As illustrated in Fig. 4, in a case where the blood purification device 3 is the initial device 3a as the information transfer source, the selection image generation unit 21a receives four pieces of transfer method data of the patient information stored in the storage unit 22 from the storage unit 22 (A in Fig. 4: transfer method data), and generates an initial selection image for allowing the operator to select the transfer method in an initial stage. In addition, the selection image generation unit 21a transmits the generated initial selection image to the display 13a (B in Fig. 4: initial selection image). Further, the selection image generation unit 21a receives, from the display 13a, initial selection information that is input by the operator of the blood purification device 3 operating the display 13a (C in Fig. 4: initial selection information). Then, the selection image generation unit 21a transmits the received initial selection information to the storage unit 22 (D in Fig. 4: initial selection information), and the initial selection information is stored in the storage unit 22.

When treatment is interrupted due to a failure or the like of the initial device 3a which is the blood purification device 3, the selection image generation unit 21a receives, from the storage unit 22, the initial selection information stored in the storage unit 22 (D in Fig. 4: initial selection information), and generates a final selection image for allowing the operator to select the transfer method at a stage of actually performing the information transfer. In addition, the selection image generation unit 21a transmits the generated final selection image to the display 13a (E in Fig. 4: final selection image). Further, the selection image generation unit 21a receives, from the display 13a, operator selection information that is input by the operator of the blood purification device 3 operating the display 13a (F in Fig. 4: operator selection information).

When the final method of the information transfer is selected, the control unit 21 receives, from the storage unit 22, the patient information stored in the storage unit 22 (G in Fig. 4: patient information). In addition, the control unit 21 executes transfer processing of the patient information based on the selected information transfer method. That is, the control unit 21 executes transfer processing of the patient information by controlling either the input/output unit 13 or the communication unit 18. Specifically, in a case where the first transfer method using the identification marker is selected, the identification marker generation unit 21b generates an identification marker corresponding to the patient information, transmits the identification marker to the display (H1 in Fig. 4: identification marker), and causes the display 13a to display the identification marker. In a case where the second transfer method using the external storage device is selected, the control unit 21 transmits the received patient information to the input/output port 13b (H2 in Fig. 4: patient information), and causes the external storage device connected to the input/output port 13b to store the patient information. In a case where the third transfer method or the fourth transfer method using the in-hospital server 4 or the cloud server 7 is selected, the control unit 21 transmits the received patient information to the transmission destination server via the communication unit 18 (H3 in Fig. 4: patient information).

As illustrated in Fig. 5, in a case where the blood purification device 3 is the alternative device 3b that is the information transfer destination, data or information necessary for performing information transfer is input. Specifically, in a case where the first transfer method using the identification marker is selected by the initial device 3a, the control unit 21 receives the identification marker which is read by the reading unit 19 (J1 in Fig. 5: identification marker). In a case where the second transfer method using the external storage device is selected by the initial device 3a, the control unit 21 reads the patient information from the external storage device connected to the input/output port 13b, and receives the patient information via the input/output port 13b (J2 in Fig. 5: patient information). In a case where the third transfer method or the fourth transfer method using the in-hospital server 4 or the cloud server 7 is selected by the initial device 3a, the control unit 21 receives the patient information, the transfer procedure method, and the difference information from the server which is a transmission source via the communication unit 18 (J3 in Fig. 5: patient information, transfer procedure method, and difference information).

The control unit 21 transmits the received information to the storage unit 22, and the patient information and the like are stored in the storage unit 22. Specifically, in a case where the first transfer method using the identification marker is selected, the calculation unit 21c acquires the patient information by performing image processing on the received identification marker. In addition, the calculation unit 21c reads past patient information stored in the storage unit 22, compares the past patient information with the patient information which is newly acquired from the identification marker, and generates difference information. Further, the calculation unit 21c also generates transfer procedure information based on the patient information and the difference information. Then, the calculation unit 21c transmits the acquired patient information, the generated difference information, and the transfer procedure information to the storage unit 22 (K in Fig. 5: patient information, transfer procedure information, difference information), and these pieces of information are stored in the storage unit 22.

In a case where the second transfer method using the external storage device is selected by the initial device 3a, the calculation unit 21c reads past patient information stored in the storage unit 22, compares the past patient information with the patient information which is received via the input/output port 13b, and generates difference information. Further, the calculation unit 21c also generates transfer procedure information based on the patient information and the difference information. Then, the calculation unit 21c transmits the acquired patient information, the generated difference information, and the transfer procedure information to the storage unit 22 (K in Fig. 5: patient information, transfer procedure information, difference information), and these pieces of information are stored in the storage unit 22.

In a case where the third transfer method or the fourth transfer method using the in-hospital server 4 or the cloud server 7 is selected by the initial device 3a, the control unit 21 transmits the received patient information, the received difference information, and the received transfer procedure information to the storage unit 22 (K in Fig. 5: patient information, transfer procedure information, and difference information), and these pieces of information are stored in the storage unit 22.

In addition, the control unit 21 transmits the patient information, the transfer procedure information, and the difference information which are received or generated to the display 13a (L in Fig. 5: patient information, transfer procedure information, difference information), and these pieces of information are displayed on the display 13a. Thereby, the operator can confirm the patient information, the transfer procedure information, and the difference information via the display 13a, and can accurately and quickly perform information transfer.

### (In-Hospital Server)

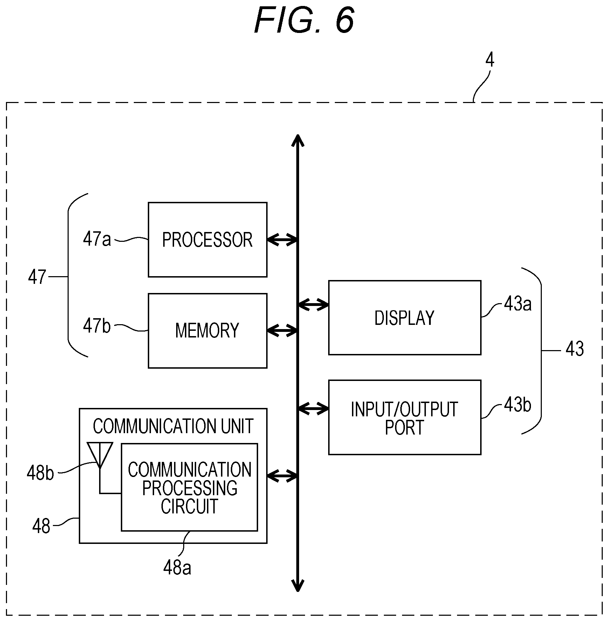

Next, an electrical configuration of the in-hospital server 4 included in the blood purification system according to the present disclosure will be described with reference to Fig. 6. Here, Fig. 6 is a block diagram illustrating an electrical configuration of the in-hospital server 4 according to the first embodiment. Note that the in-hospital server 4 does not need to include all of the components illustrated in Fig. 6. Some of the components can be omitted, or another component can be added.

As illustrated in Fig. 6, the in-hospital server 4 includes an input/output unit 43 including a display 43a and an input/output port 43b, an information processing device 47 including a processor 47a and a memory 47b, and a communication unit 48 including a communication processing circuit 48a and an antenna 48b. Then, these components are electrically connected to each other via a control line and a data line.

The display 43a has a general screen-type output interface. That is, the display 43a in the present embodiment has only an output function unlike the display 13a of the blood purification device 3. Note that the display 43a may be a touch panel including an input/output interface, similarly to the display 13a of the blood purification device 3.

Similarly to the input/output port 13b of the blood purification device 3, the input/output port 43b is configured with terminals of the interface used for input/output of information and data with various devices connected to the outside of the in-hospital server 4. That is, the input/output port 43b can be connected to an external storage device (not illustrated), and has an input/output function that enables input/output of information and data between the in-hospital server 4 and the external storage device. For example, the external storage device that can be connected to the input/output port 43b may be a USB memory, a CF card, an NFC card, or the like. Note that the in-hospital server 4 may not include the input/output port 43b in a case where an external storage device or the like is not connected.

The processor 47a is configured with a GPU or a CPU, and functions as a control unit for controlling another device directly connected or another device connected via a communication line based on various programs stored in the memory 47b. For example, the processor 47a reads a program for executing information processing according to the present disclosure or a program for executing an OS function from the memory 47b, and executes the program. More specifically, the processor 47a executes processing such as processing and transmission of the received patient information in order to execute processing related to transfer of the patient information. In particular, similarly to the control unit 21 of the blood purification device 3, the processor 47a generates transfer procedure information and difference information based on the received latest patient information and the past patient information stored in the memory 47b. Details of these pieces of processing will be described later. Note that the processor 47a may be configured with a single CPU, or may be configured with a plurality of CPUs.

The memory 47b includes a RAM, a ROM, a nonvolatile memory, and an HDD, and functions as a storage unit. The ROM stores, as a program, instruction commands for executing information processing and an OS function according to the present disclosure. Such a program is loaded and executed by the processor 47a. The RAM is used to write and read data while the program stored in the ROM is being processed by the processor 47a. The nonvolatile memory is a memory in which writing and reading of data are executed by execution of the program, and the data written in the nonvolatile memory is stored even after the execution of the program is completed. In the present disclosure, in particular, the memory 47b stores a program for executing processing such as processing and transmission of received patient information in order to execute processing related to transfer of patient information.

In addition, the memory 47b stores patient information and the like related to treatments of a plurality of patients. Similarly to the storage unit 22 of the blood purification device 3, the memory 47b also stores transfer procedure information, which is a procedure related to transfer of patient information, and difference information between new patient information and past patient information.

The communication unit 48 transmits and receives information to and from the blood purification device 3 which is installed in the hospital or the mobile terminal 5, or the cloud server 7 which is installed at a remote location via the communication processing circuit 48a and the antenna 48b. The communication processing circuit 48a performs processing for transmitting and receiving information to and from the blood purification device 3, the mobile terminal 5, or the cloud server 7 according to the progress of the processing, the information including programs, various types of information, and the like that are to be used in the blood purification system 1.

Note that the communication processing circuit 48a performs processing based on a broadband wireless communication system typified by an LTE system, but can also perform processing based on a system related to narrowband wireless communication such as a wireless LAN typified by IEEE 802.11 or Bluetooth (registered trademark) or a system related to non-contact wireless communication. In addition, wired communication can be used instead of or in addition to wireless communication.

### (Mobile Terminal)

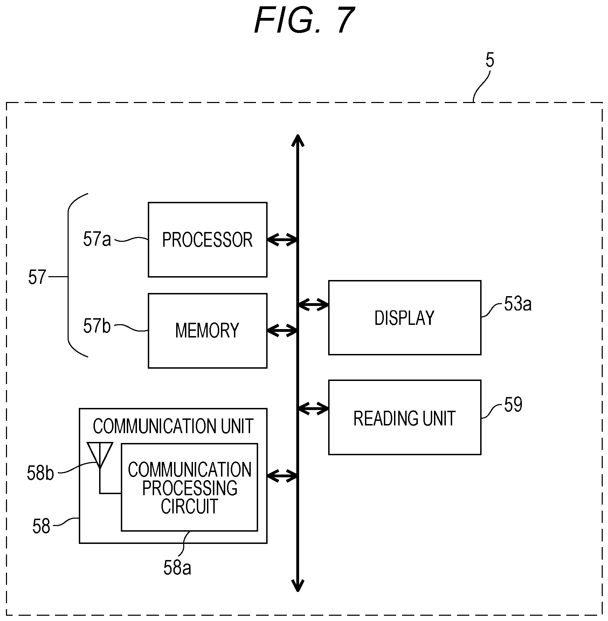

Next, an electrical configuration of the mobile terminal 5 included in the blood purification system according to the present disclosure will be described with reference to Fig. 7. Here, Fig. 7 is a block diagram illustrating an electrical configuration of the mobile terminal 5 according to the first embodiment. Note that the mobile terminal 5 does not need to include all of the components illustrated in Fig. 7. Some of the components can be omitted, or another component can be added.

As illustrated in Fig. 7, the mobile terminal 5 includes a display 53a, an information processing device 57 including a processor 57a and a memory 57b, a communication unit 58 including a communication processing circuit 58a and an antenna 58b, and a reading unit 59. Then, these components are electrically connected to each other via a control line and a data line.

Next, the display 53a includes a touch-panel-type input interface and a general screen-type output interface. That is, the display 53a in the present embodiment is a touch panel including an input/output interface, and has an input function and an output function. Here, the input detection method by the touch panel may be any method such as a capacitance type or a resistive film type. In particular, in the present embodiment, the display 13a displays patient information acquired from the blood purification device 3, or displays an identification marker read from the blood purification device 3.

The processor 57a is configured with a GPU or a CPU, and functions as a control unit for controlling each component of the mobile terminal 5 based on various programs stored in the memory 57b. For example, the processor 57a reads a program for executing information processing according to the present disclosure or a program for executing an OS function from the memory 57b, and executes the program. More specifically, the processor 57a executes reading of the identification marker by the reading unit 59, information processing related to acquisition of the patient information and the like from the read identification marker, processing of displaying the patient information and the like on the display 53a, and processing of displaying the identification marker on the display 53a in order to execute processing related to transfer of the patient information. Details of these pieces of processing will be described later. Note that the processor 57a may be configured with a single GPU or CPU, or may be configured with a plurality of GPUs or CPUs.

The memory 57b includes a RAM, a ROM, a nonvolatile memory, and an HDD, and functions as a storage unit. The ROM stores, as a program, instruction commands for executing information processing and an OS function according to the present disclosure. Such a program is loaded and executed by the processor 57a. The RAM is used to write and read data while the program stored in the ROM is being processed by the processor 57a. The nonvolatile memory is a memory in which writing and reading of data are executed by execution of the program, and the data written in the nonvolatile memory is stored even after the execution of the program is completed. In the present disclosure, in particular, the memory 57b stores programs for executing reading of the identification marker by the reading unit 59, information processing related to acquisition of the patient information and the like from the read identification marker, processing of displaying the patient information and the like on the display 53a, and processing of displaying the identification marker on the display 53a in order to execute processing related to transfer of the patient information.

The communication unit 58 transmits and receives information to and from the blood purification device 3 or the in-hospital server 4 which is installed in the hospital or the cloud server 7 which is installed at a remote location via the communication processing circuit 58a and the antenna 58b. The communication processing circuit 58a performs processing for transmitting and receiving information to and from the blood purification device 3, the in-hospital server 4, or the cloud server 7 according to the progress of the processing, the information including programs, various types of information, and the like that are to be used in the blood purification system 1.

Note that the communication processing circuit 58a performs processing based on a broadband wireless communication system typified by an LTE system, but can also perform processing based on a system related to narrowband wireless communication such as a wireless LAN or Bluetooth (registered trademark) typified by IEEE 802.11 or a system related to non-contact wireless communication. In addition, wired communication can be used instead of or in addition to wireless communication.

The reading unit 59 is a general camera, and is an information acquisition device capable of reading a one-dimensional or two-dimensional identification marker. In the present embodiment, the reading unit 59 reads the identification marker to be displayed on the display 13a of the blood purification device 3 (the initial device 3a). For example, similarly to the reading unit 19 of the blood purification device 3, the reading unit 59 optically detects the identification marker, and converts the identification marker into specific information (patient information) by analyzing patterns of a black portion and a white portion (a blank portion).

Note that, in the present embodiment, the identification marker is read and displayed using the display 53a and the reading unit 59 of the mobile terminal 5, but the present disclosure is not limited thereto. That is, a dedicated reading terminal for reading and displaying the identification marker may be prepared separately from the mobile terminal 5, and may be used as a constituent device of the blood purification system 1.

### (Cloud Server)

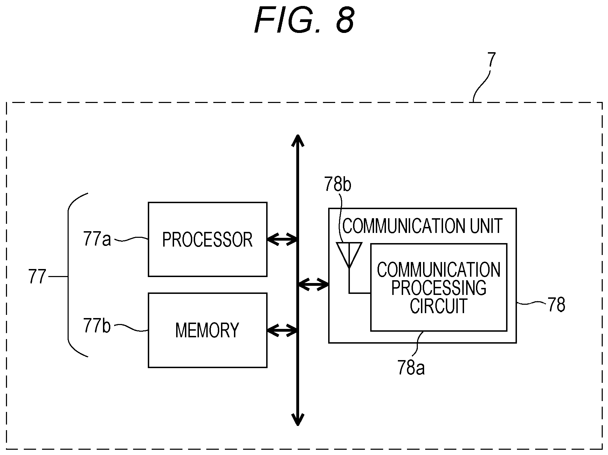

Next, an electrical configuration of the cloud server 7 included in the blood purification system according to the present disclosure will be described with reference to Fig. 8. Here, Fig. 8 is a block diagram illustrating an electrical configuration of the cloud server 7 according to the first embodiment. Note that the cloud server 7 does not need to include all of the components illustrated in Fig. 8. Some of the components can be omitted, or another component can be added.

As illustrated in Fig. 8, the cloud server 7 includes an information processing device 77 including a processor 77a and a memory 77b, and a communication unit 78 including a communication processing circuit 78a and an antenna 78b. Then, these components are electrically connected to each other via a control line and a data line.

The processor 77a is configured with a GPU or a CPU, and functions as a control unit for controlling another device directly connected or another device connected via a communication line based on various programs stored in the memory 77b. For example, the processor 77a reads a program for executing information processing according to the present disclosure or a program for executing an OS function from the memory 77b, and executes the program. More specifically, the processor 77a executes processing such as processing and transmission of the received patient information in order to execute processing related to transfer of the patient information. In particular, similarly to the control unit 21 of the blood purification device 3, the processor 77a generates transfer procedure information and difference information based on the received latest patient information and the past patient information stored in the memory 77b. Details of these pieces of processing will be described later. Note that the processor 77a may be configured with a single GPU or CPU, or may be configured with a plurality of GPUs or CPUs.

The memory 77b includes a RAM, a ROM, a nonvolatile memory, and an HDD, and functions as a storage unit. The ROM stores, as a program, instruction commands for executing information processing and an OS function according to the present disclosure. Such a program is loaded and executed by the processor 77a. The RAM is used to write and read data while the program stored in the ROM is being processed by the processor 77a. The nonvolatile memory is a memory in which writing and reading of data are executed by execution of the program, and the data written in the nonvolatile memory is stored even after the execution of the program is completed. In the present disclosure, in particular, the memory 77b stores a program for executing processing such as processing and transmission of received patient information in order to execute processing related to transfer of patient information.

In addition, the memory 77b stores patient information related to treatments of a plurality of patients. Similarly to the storage unit 22 of the blood purification device 3, the memory 77b also stores transfer procedure information, which is a procedure related to transfer of patient information, and difference information between new patient information and past patient information.

The communication unit 78 transmits and receives information to and from the blood purification device 3 which is installed at a remote location, the in-hospital server 4, or the mobile terminal 5 via the communication processing circuit 78a and the antenna 78b. The communication processing circuit 78a performs processing for transmitting and receiving information to and from the blood purification device 3, the in-hospital server 4, or the mobile terminal 5 according to the progress of the processing, the information including programs, various types of information, and the like that are to be used in the blood purification system 1.

Note that the communication processing circuit 78a performs processing based on a broadband wireless communication system typified by an LTE system, but can also perform processing based on a system related to narrowband wireless communication such as a wireless LAN or Bluetooth (registered trademark) typified by IEEE 802.11 or a system related to non-contact wireless communication. In addition, wired communication can be used instead of or in addition to wireless communication.

### (Processing in Blood Purification System)

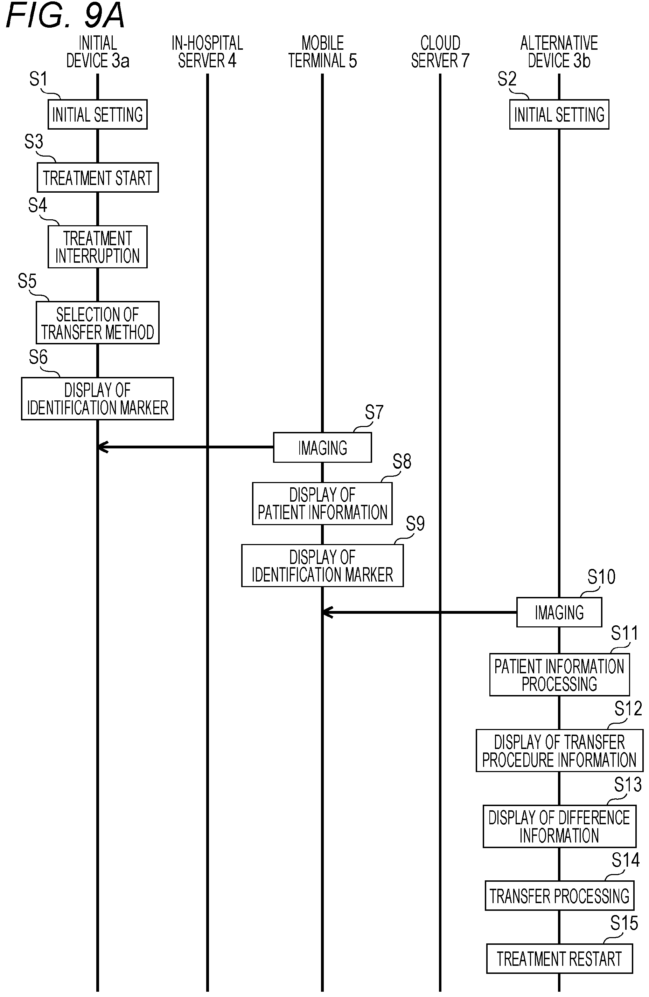

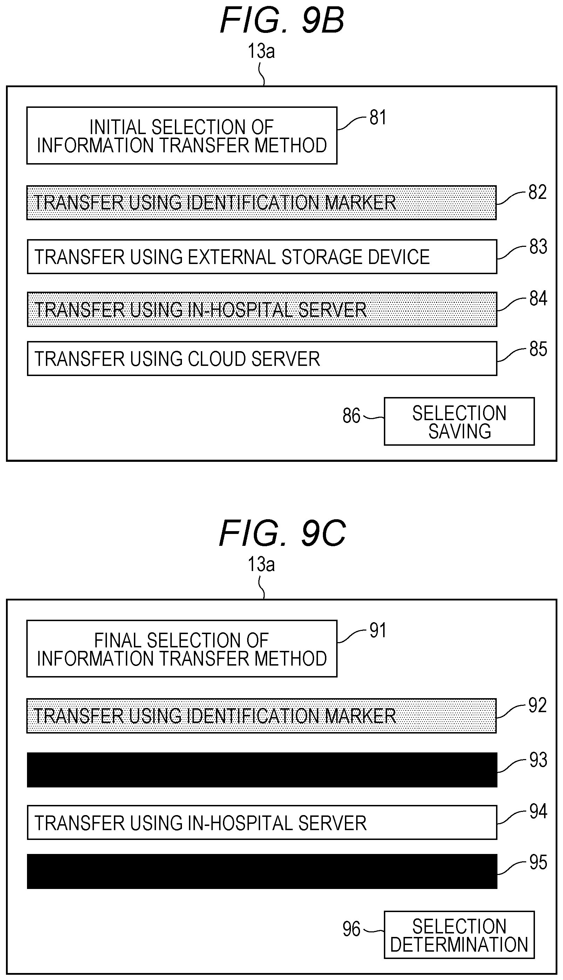

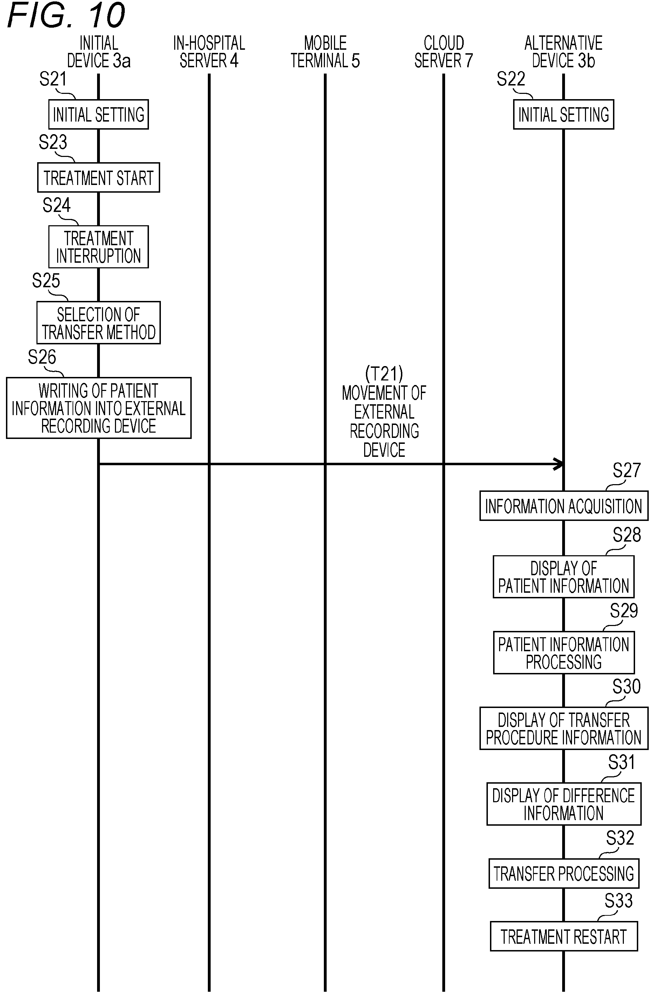

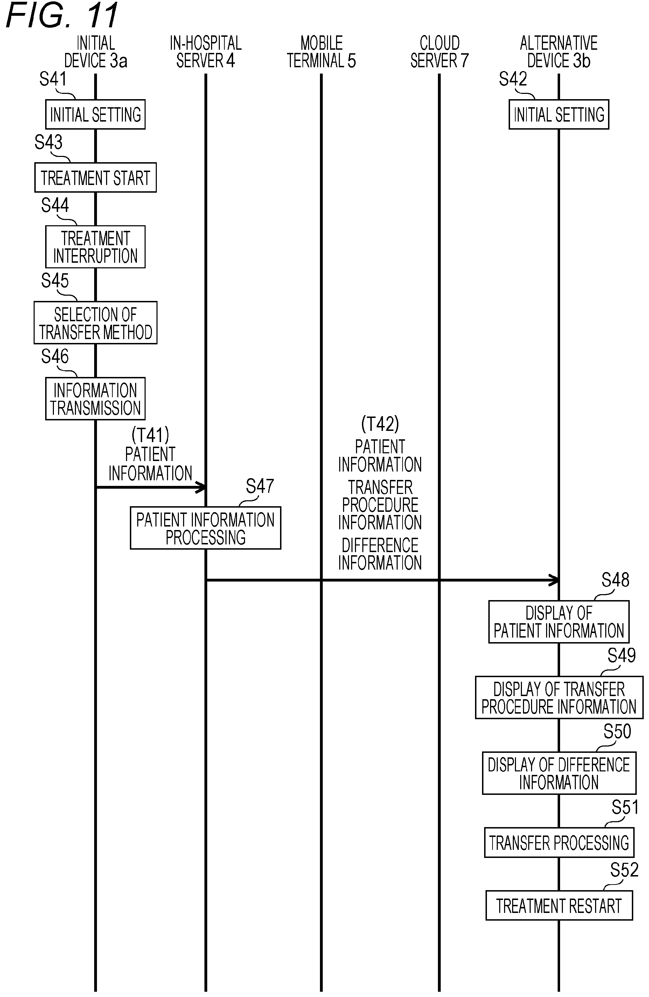

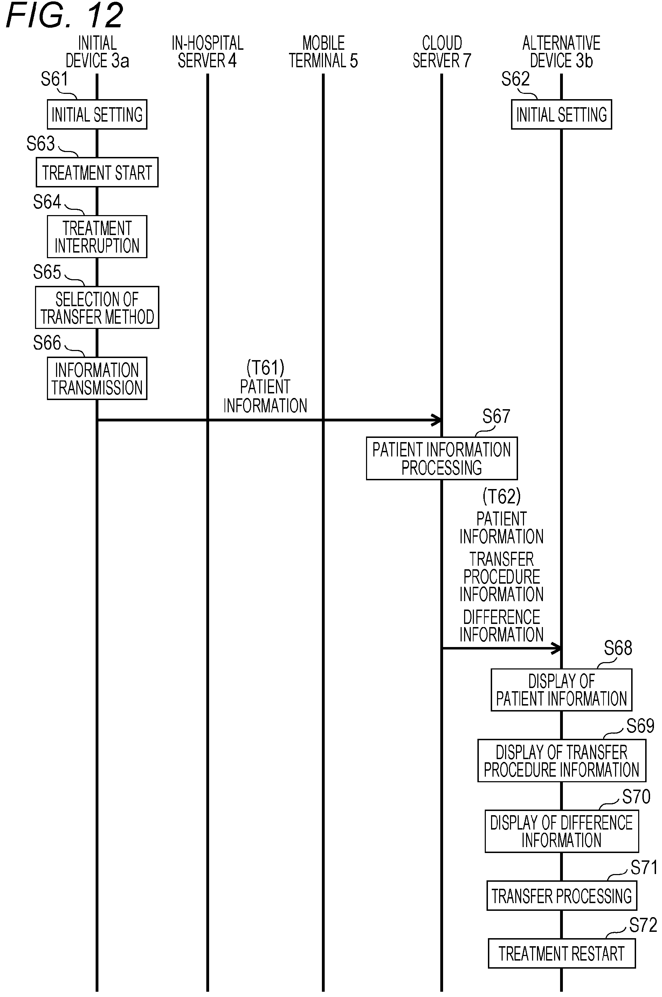

Next, each of the first to fourth transfer methods will be described with reference to Fig. 9A to Fig. 12 with regard to the processing related to transfer of the patient information in the blood purification system 1 according to the present embodiment. Here, Fig. 9A and Fig. 10 to Fig. 12 are sequence diagrams related to information transfer in the blood purification system 1 according to the present embodiment. Fig. 9B to Fig. 9H are examples of screens displayed in the blood purification device 3 according to the present embodiment. In particular, Fig. 9A is a sequence diagram in a case where the first transfer method is selected, Fig. 10 is a sequence diagram in a case where the second transfer method is selected, Fig. 11 is a sequence diagram in a case where the third transfer method is selected, and Fig. 12 is a sequence diagram in a case where the fourth transfer method is selected.

### [First Transfer Method]

First, in the first transfer method, as illustrated in Fig. 9A, initial setting related to information transfer is performed in each of the blood purification devices 3 (the initial device 3a and the alternative device 3b) (S1 and S2). The initial setting is performed in each of the blood purification devices 3 by the operator of the blood purification device 3 when the blood purification device 3 is introduced into the hospital. Specifically, the information processing device 17 of the blood purification device 3 displays the initial selection information on the display 13a, and allows the operator to select the initial selection information. For example, as illustrated in Fig. 9B, the display 13a displays a title display portion 81 in which an operation title of "initial selection of information transfer method" is described, a first selection button 82 in which "transfer using identification marker" as the first transfer method is displayed, a second selection button 83 in which "transfer using external storage device" as the second transfer method is displayed, a third selection button 84 in which "transfer using in-hospital server" as the third transfer method is displayed, a fourth selection button 85 in which "transfer using cloud server", as the fourth transfer method is displayed, and a saving button 86 in which "selection saving" for saving the selected transfer method is displayed.

Here, in Fig. 9B, the first selection button 82 and the third selection button 84 are pressed by the operator, the first transfer method and the third transfer method are selected, and the color of each button is displayed in a darker color. Then, when the saving button 86 is pressed by the operator, the first transfer method and the third transfer method are transmitted to the control unit 21 as initial selection information. Thereafter, the control unit 21 transmits the initial selection information to the storage unit 22 (the memory 17b), and the initial selection information is stored in the storage unit 22.

Next, treatment is started using the blood purification device 3 (the initial device 3a) (S3). Specifically, the operator connects the accessories to the blood purification device 3, prepares a medication agent, and performs puncture on the patient. Further, setting of treatment conditions corresponding to the patient is performed, and treatment corresponding to each patient is started. Note that data related to the treatment which is newly performed is appropriately stored in the storage unit 22 as the patient information by processing of the control unit 21.

Thereafter, one blood purification device 3 fails due to some reason, and the treatment in the initial device 3a as the blood purification device 3 is interrupted (S4). When the control unit 21 of the initial device 3a detects the interruption of the treatment, processing of selecting the transfer method in the initial device 3a is performed (S5). Specifically, the control unit 21 reads the initial selection information from the storage unit 22, and the selection image generation unit 21a generates a final selection image. The final selection image is transmitted to the display 13a, and is displayed on the display 13a.

For example, as illustrated in Fig. 9C, the display 13a displays a title display portion 91 in which an operation title of "final selection of information transfer method" is described, a first selection button 92 in which "transfer using identification marker" as the first transfer method is displayed, a second selection button 93 in which "transfer using external storage device" as the second transfer method is displayed, a third selection button 94 in which "transfer using in-hospital server" as the third transfer method is displayed, a fourth selection button 95 in which "transfer using cloud server" as the fourth transfer method is displayed, and a determination button 96 in which "selection determination" for determining the selected transfer method is displayed.

Here, in the present embodiment, since the second and fourth transfer methods are not selected at the initial selection stage, the second selection button 93 and the fourth selection button 95 are displayed in a black state so as not to be selected by the operator. Note that the second selection button 93 and the fourth selection button 95 according to the second and fourth transfer methods which are not selected at the initial selection stage may be hidden on the display 13a such that the operator cannot view and select the buttons. On the other hand, the second selection button 93 and the fourth selection button 95 may be displayed to be selectable in order to expand the options in this stage.

Then, in Fig. 9C, the first selection button 92 is pressed by the operator, the first transfer method is selected, and the color of the first selection button 92 is displayed in a darker color. Thereafter, when the determination button 96 is pressed by the operator, operator selection information, which is final selection information and indicates selection of the first transfer method, is transmitted to the control unit 21.

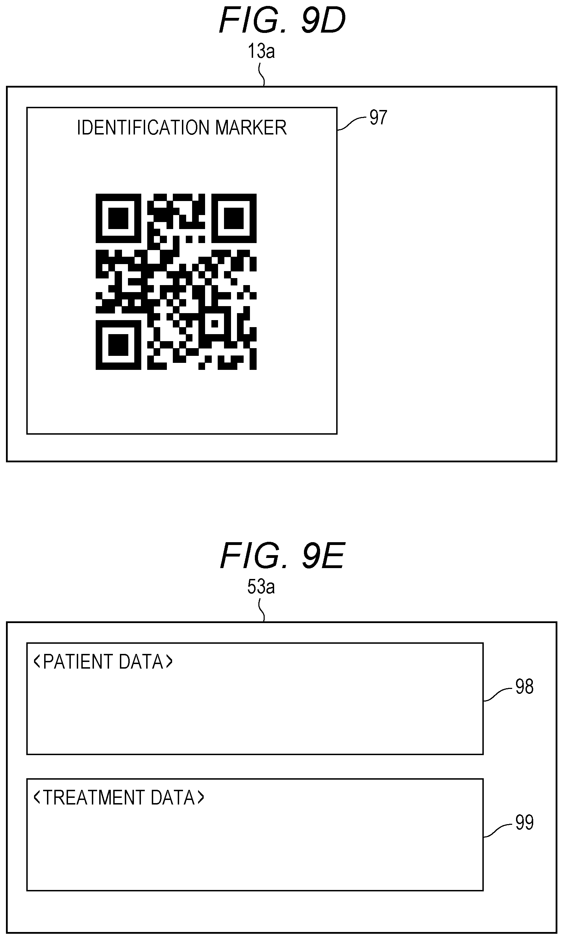

Next, in the initial device 3a, the identification marker is displayed on the display 13a (S6). Specifically, the identification marker generation unit 21b of the control unit 21 reads the patient information from the storage unit 22 when receiving the operator selection information. The patient information includes specific data of a patient who has received treatment in the initial device 3a and treatment data, and the treatment data also includes treatment progress data from a start of the treatment to an interruption of the treatment. That is, the patient information includes information necessary for take-over of the treatment from the initial device 3a to the alternative device 3b. Then, the identification marker generation unit 21b generates a one-dimensional or two-dimensional identification marker corresponding to the patient information, and transmits the identification marker to the display 13a to display the identification marker. For example, as illustrated in Fig. 9D, a two-dimensional QR code (registered trademark) may be displayed as the identification marker on a marker display portion 97 of the display 13a.

Next, the identification marker displayed on the display 13a of the initial device 3a is imaged by an operation of the mobile terminal 5 (S7). Specifically, in the mobile terminal 5 owned by the operator of the blood purification device 3, the operator operates the screen displayed on the display 53a, and an imaging program of the identification marker by the information processing device 57 of the mobile terminal 5 is executed. Then, the operation of the reading unit 59 of the mobile terminal 5 is controlled, and the identification marker displayed on the display 13a of the initial device 3a is imaged.

Next, the information processing device 57 of the mobile terminal 5 acquires patient information by performing image processing on the imaged identification marker, and displays the patient information on the display 53a (S8). For example, as illustrated in Fig. 9E, on the display 53a, patient-specific data that is one piece of patient information may be displayed in a display portion 98, and treatment data that is another piece of patient information may be displayed in a display portion 99. In the display portion 98, the name, the gender, the weight, the height, and the blood pressure of the patient that are described above may be displayed, and in the display portion 99, the data on treatment conditions, the data on medical materials, the data on accessories attached to the blood purification device, the data on a medication agent, and the treatment progress data of the patient that are described above may be displayed. Thereby, the operator of the blood purification device 3 can easily and quickly confirm the information on the patient whose treatment is interrupted, the treatment situation of the patient, and the like.

Next, the same identification marker as the identification marker displayed on the display 13a of the initial device 3a is displayed on the display 53a of the mobile terminal 5 (S9). Specifically, in the mobile terminal 5 owned by the operator, when the operator operates the screen displayed on the display 53a, a program for redisplaying the identification marker by the information processing device 57 of the mobile terminal 5 is executed.

Next, the identification marker displayed on the display 53a is imaged by the operation of the alternative device 3b as the information transfer destination (S10). Specifically, when the operator of the blood purification device 3 operates the screen displayed on the display 13a of the alternative device 3b, an imaging program of the identification marker by the information processing device 17 of the alternative device 3b is executed. Then, the operation of the reading unit 19 of the alternative device 3b is controlled, and the identification marker displayed on the display 53a of the mobile terminal 5 is imaged.

Next, the information processing device 17 of the alternative device 3b acquires patient information by performing image processing on the imaged identification marker, and performs information processing on the patient information (S11). Specifically, the calculation unit 21c functioning by the information processing device 17 of the alternative device 3b acquires patient information by performing image processing on the image of the identification marker that is received from the reading unit 19, and executes installation of the patient information. Thereby, the alternative device 3b can take over the patient information of the initial device 3a, and can perform quick transfer processing using the patient information of the initial device 3a. In addition, the calculation unit 21c reads past patient information stored in the storage unit 22, extracts a difference between the past patient information and patient information which is newly acquired from the identification marker by comparing the past patient information with the newly-acquired patient information, and generates difference information related to the patient information. Further, the calculation unit 21c also generates transfer procedure information indicating a transfer procedure of the patient information in the alternative device 3b based on the newly-acquired patient information and the generated difference information.

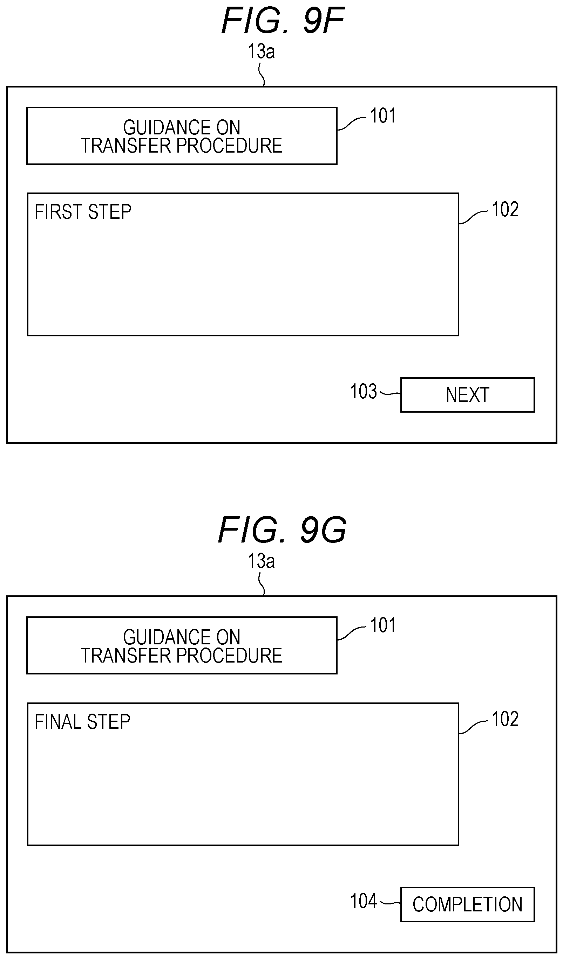

Next, in the alternative device 3b, the transfer procedure information generated by the patient information processing is displayed on the display 13a (S12). Specifically, the control unit 21 of the alternative device 3b transmits the transfer procedure information to the display 13a, and displays the transfer procedure information on the display 13a such that the operator can confirm the transfer procedure information. For example, as illustrated in Fig. 9F, the display 13a displays a title display portion 101 in which an operation title of "guidance of transfer procedure" is described, a transfer procedure display portion 102 in which "first step" which is processing content at an initial stage of the transfer procedure is displayed, and a transition button 103 in which "next" for proceeding to the next step is displayed. Then, when the transition button 103 is pressed by the operator, the transfer procedure sequentially proceeds. As illustrated in Fig. 9G, when the final step is displayed in the transfer procedure display portion 102, a completion button 104 in which "completion" is displayed and which causes the control unit 21 to recognize that the operation of the transfer procedure has been completed is displayed in a lower portion of the display 13a.

Note that the display related to the guidance of the transfer procedure is not limited to the above content, and all steps may be displayed at the same time, or the process may proceed to the next step by scrolling the screen. In addition, another transition button in which "return" is displayed may be displayed such that the operator can reconfirm the content by returning the process to the previous step.

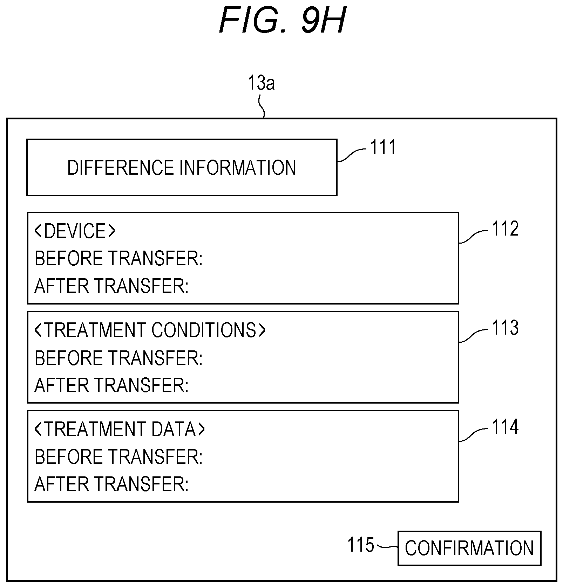

Next, in the alternative device 3b, the difference information generated by the patient information processing is displayed on the display 13a (S13). Specifically, the control unit 21 of the alternative device 3b transmits the difference information to the display 13a, and displays the difference information on the display 13a such that the operator can confirm the difference information. For example, as illustrated in Fig. 9H, the display 13a displays a title display portion 111 in which a title of "difference information" is described, a difference display portion 112 in which the difference information related to the device is displayed, a difference display portion 113 in which the difference information related to the treatment conditions is displayed, a difference display portion 114 in which the difference information related to the treatment data is displayed, and a confirmation button 115 in which "confirmation" is displayed and which causes the control unit 21 to recognize completion of confirmation of the difference. In each of the difference display portions, information which is stored in the initial device 3a and is information before the transfer is displayed, and information which is stored in the alternative device 3b and is information after the transfer is displayed. Thereby, the operator can easily recognize the content to be noted in the transfer processing, and thus, transfer processing can be quickly performed.

Note that the order of the display of the transfer procedure information in S12 and the display of the difference information in S13 may be changed. In addition, the two pieces of information may be displayed such that the two pieces of information can be simultaneously confirmed.

Next, in the alternative device 3b, various types of transfer processing are performed by the operator (S14). Specifically, setting of the treatment conditions, attaching accessories, preparation of the medication agent, movement of the patient, and the like are performed. Note that the operator may confirm the display of the transfer procedure information in S12 and the display of the difference information in S13, and may determine to end the treatment without restarting the treatment in a case where there is a reason such as a case where the transfer processing cannot be quickly performed. For example, in a case where transfer cannot be quickly performed, such as a case where there is no accessory to be attached to the alternative device 3b or a case where it takes time to attach an accessory, the treatment is terminated.

Next, in the alternative device 3b, the treatment is restarted by processing by the operator (S15). Specifically, when the operator operates the screen displayed on the display 13a of the alternative device 3b, an operation of the alternative device 3b that is related to the treatment is started.

Note that, in the first transfer method, the transfer processing of the patient information is performed via the mobile terminal 5, but the transfer processing of the patient information may be performed without passing through the mobile terminal 5. That is, the reading unit 19 of the alternative device 3b may read the identification marker displayed on the display 13a of the initial device 3a, and the processing of the calculation unit 21c of the alternative device 3b may be performed. In addition, the reason why the treatment is interrupted is not limited to a failure of the initial device 3a, and may be due to circumstances of the hospital 2 or the patient.

### [Second Transfer Method]

The second transfer method is different from the first transfer method in that the patient information is transferred using an external storage device and that the patient information is displayed on the alternative device 3b instead of the mobile terminal 5. Therefore, focusing on a point different from the first transfer method, a processing flow will be described with reference to Fig. 10, and the description of the same content as the first transfer method will be omitted or simplified.

First, similarly to the initial setting (S1, S2), the treatment start (S3), the treatment interruption (S4), and the selection of the transfer method (S5) in the first transfer method, initial setting (S21, S22), treatment start (S23), treatment interruption (S24), and selection of the transfer method (S25) are performed. Thereafter, writing of the patient information into an external recording device is performed (S26). Specifically, when the operator of the initial device 3a connects the external recording device to the input/output port of the initial device 3a, the control unit 21 of the initial device 3a recognizes the external recording device. Thereafter, the control unit 21 of the initial device 3a reads the patient information from the storage unit 22, and records the patient information in the external recording device via the input/output port 13b.

Next, the operator of the initial device 3a moves the external recording device to the alternative device 3b (T21), and the external storage device is connected to the input/output port 13b of the alternative device 3b. Thereby, the control unit 21 of the alternative device 3b recognizes the external recording device, and the control unit 21 acquires the patient information from the external recording device (S27).

Next, similarly to the display of the patient information in the first transfer method (S8), the patient information which is acquired in the alternative device 3b is displayed on the display 13a (S28). That is, the operator confirms the specific data and the treatment data in the alternative device 3b that is the transfer destination of the patient information, and recognizes the content of the specific data and the treatment data.

Next, similar to the patient information processing (S11), the display of the transfer procedure information (S12), the display of the difference information (S13), the transfer processing (S14), and the treatment restart (S15) in the first transfer method, patient information processing (S29), display of the transfer procedure information (S30), display of the difference information (S31), transfer processing (S32), and treatment restart (S33) are performed.

Note that, in the second transfer method, the information transfer is performed by connecting the external storage device to the input/output port of the alternative device 3b. However, the external storage device may be connected to the input/output port 43b of the in-hospital server 4. In this case, after the patient information is processed in the in-hospital server 4, the patient information, the difference information, and the transfer procedure information may be transmitted to the alternative device 3b via a communication line.

### [Third Transfer Method]

The third transfer method is different from the first and second transfer methods in that the patient information is transferred via the in-hospital server 4 using wired communication or wireless communication, and that patient information processing can be performed in the in-hospital server 4 instead of the alternative device 3b. Therefore, focusing on a point different from the first and second transfer methods, a processing flow will be described with reference to Fig. 11, and the description of the same content as the first and second transfer methods will be omitted or simplified.

First, similarly to the initial setting (S1, S2), the treatment start (S3), the treatment interruption (S4), and the selection of the transfer method (S5) in the first transfer method, initial setting (S41, S42), treatment start (S43), treatment interruption (S44), and selection of the transfer method (S45) are performed. Thereafter, transmission processing of the patient information is performed in the initial device 3a (S46), and the patient information is transmitted to the in-hospital server 4 (T41). Specifically, the control unit 21 of the initial device 3a transmits the patient information which is read from the storage unit 22 to the in-hospital server 4 as a transmission destination via the communication unit 18.

Next, similarly to the blood purification device 3, the information processing device 47 of the in-hospital server 4 performs information processing on the received patient information (S47). Specifically, the processor 47a of the in-hospital server 4 reads past patient information stored in the memory 47b, extracts a difference by comparing the past patient information with newly-received patient information, and generates difference information related to the patient information. Further, the processor 47a also generates transfer procedure information indicating a transfer procedure of the patient information in the alternative device 3b based on the newly-received patient information and the generated difference information.

Next, the information processing device 47 of the in-hospital server 4 transmits the patient information, the difference information, and the transfer procedure information to the alternative device 3b via the communication unit 48 (T42). Thereby, the control unit 21 of the alternative device 3b receives the patient information via the communication unit 18.

Next, similarly to the display of the patient information in the second transfer method (S28), the patient information received by the alternative device 3b is displayed on the display 13a (S48). That is, the operator confirms the specific data and the treatment data in the alternative device 3b that is the transfer destination of the patient information, and recognizes the content of the specific data and the treatment data.

Next, similarly to the display of the transfer procedure information (S12), the display of the difference information (S13), the transfer processing (S14), and the treatment restart (S15) in the first transfer method, display of the transfer procedure information (S49), display of the difference information (S50), transfer processing (S51), and treatment restart (S52) are performed.

### [Fourth Transfer Method]

The fourth transfer method is different from the first and second transfer methods in that the patient information is transferred via the cloud server 7 using wired communication or wireless communication, and that patient information processing can be performed in the cloud server 7 instead of the alternative device 3b. Therefore, focusing on a point different from the first and second transfer methods, a processing flow will be described with reference to Fig. 12, and the description of the same content as the first and second transfer methods will be omitted or simplified.

First, similarly to the initial setting (S1, S2), the treatment start (S3), the treatment interruption (S4), and the selection of the transfer method (S5) in the first transfer method, initial setting (S61, S62), treatment start (S63), treatment interruption (S64), and selection of the transfer method (S65) are performed. Thereafter, transmission processing of the patient information is performed in the initial device 3a (S66), and the patient information is transmitted to the cloud server 7 (T61). Specifically, the control unit 21 of the initial device 3a transmits the patient information which is read from the storage unit 22 to the cloud server 7 as a transmission destination via the communication unit 18.

Next, similarly to the blood purification device 3, the information processing device 77 of the cloud server 7 performs information processing on the received patient information (S67). Specifically, the processor 77a of the cloud server 7 reads past patient information stored in the memory 77b, extracts a difference by comparing the past patient information with newly-received patient information, and generates difference information related to the patient information. Further, the processor 77a also generates transfer procedure information indicating a transfer procedure of the patient information in the alternative device 3b based on the newly-received patient information and the generated difference information.

Next, the information processing device 77 of the cloud server 7 transmits the patient information, the difference information, and the transfer procedure information to the alternative device 3b via the communication unit 78 (T62). Thereby, the control unit 21 of the alternative device 3b receives the patient information via the communication unit 18.

Next, similarly to the display of the patient information in the second transfer method (S28), the patient information received by the alternative device 3b is displayed on the display 13a (S68). That is, the operator confirms the specific data and the treatment data in the alternative device 3b that is the transfer destination of the patient information, and recognizes the content of the specific data and the treatment data.

Next, similarly to the display of the transfer procedure information (S12), the display of the difference information (S13), the transfer processing (S14), and the treatment restart (S15) in the first transfer method, display of the transfer procedure information (S69), display of the difference information (S70), transfer processing (S71), and treatment restart (S72) are performed.

### (Operations and Effects of First Embodiment)

In the present embodiment, four types of transfer methods for patient information transfer are stored in the information processing device 17 of the blood purification device 3, and display or the like is performed such that the operator can select the transfer method at an initial stage and a stage immediately before the information transfer. That is, according to the environments or the like of the blood purification device 3 and the blood purification system 1, any one of the transfer methods in which the used devices are different is selected and information transfer is performed.

Therefore, it is possible to adopt an optimal transfer method corresponding to the states of the blood purification device 3 and the blood purification system 1 or the installation environments of the blood purification device 3 and the blood purification system 1, and it is possible to easily and accurately perform information transfer and shorten a lead time until the treatment is restarted. In particular, in the present embodiment, since the operator selects the transfer method, it is easy to reflect an actual situation of the hospital 2, and thus, information transfer can be performed with higher accuracy. In addition, since it is not necessary to input, to the blood purification device 3, various types of information related to the patient and the transfer of the blood purification device 3, a human error such as an input error does not occur, and the overall processing time can be reduced.

### <Second Embodiment>

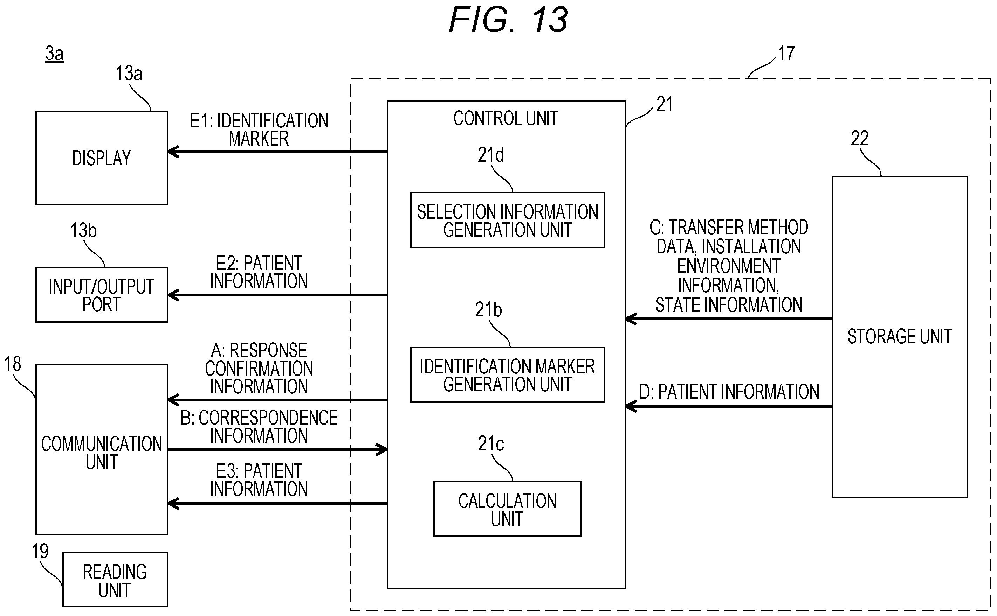

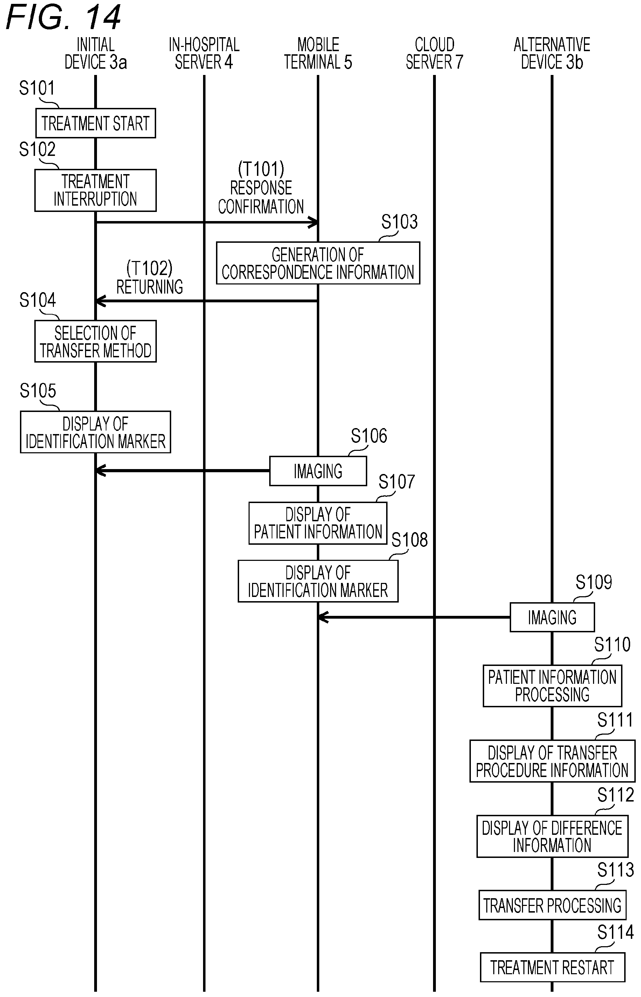

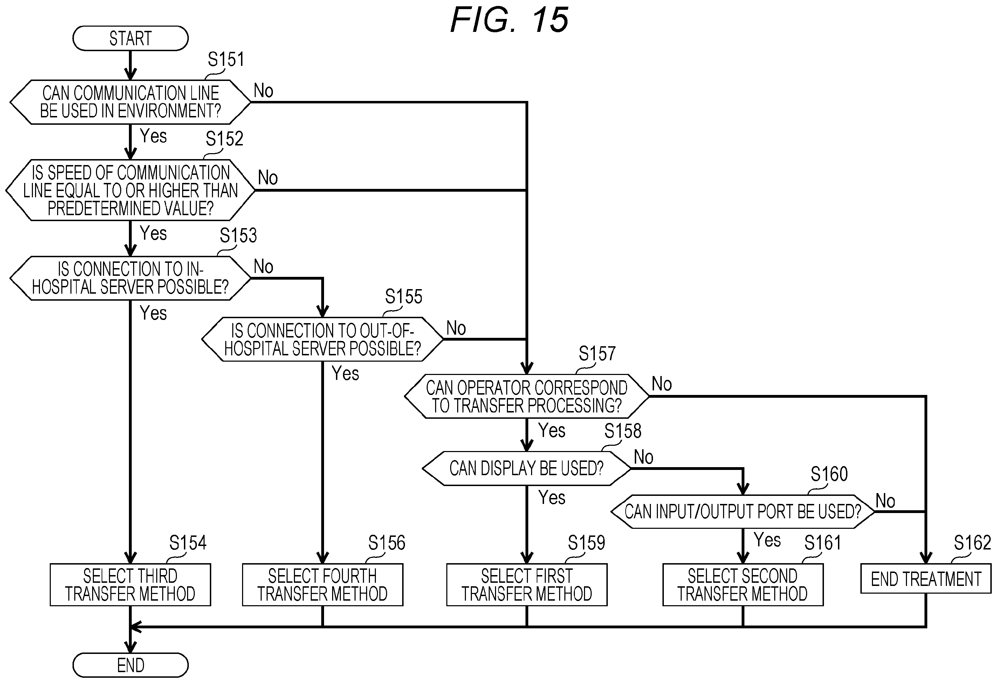

In the first embodiment, the operator of the blood purification device 3 selects the patient information transfer method twice, but the blood purification device 3 may automatically select the transfer method based on predetermined information. That is, the control unit 21 of the blood purification device 3 may generate the control unit selection information, any one of the first to fourth transfer methods may be selected based on the control unit selection information, and the transfer processing of the patient information may be performed. Such a case will be described as a second embodiment with reference to Fig. 13 to Fig. 15. Here, Fig. 13 is a functional block diagram related to information transfer of the blood purification device 3 (the initial device 3a) according to the second embodiment. Fig. 14 is a sequence diagram related to information transfer in the blood purification system 1 according to the second embodiment. Fig. 15 is a flowchart related to selection of a transfer method in the blood purification device 3 (the initial device 3a) according to the second embodiment. Note that parts different from the first embodiment will be basically described, description of the same content will be omitted, and the same reference numerals will be basically used in the drawings.

First, as illustrated in Fig. 13, the control unit 21 of the information processing device 17 includes an identification marker generation unit 21b, a calculation unit 21c, and a selection information generation unit 21d. That is, unlike the first embodiment, the control unit 21 does not include the selection image generation unit 21a that generates an image to be selected by the operator, but includes the selection information generation unit 21d that generates selection information for selecting a transfer method. Similarly to the first embodiment, these units are implemented by causing the processor 17a and the memory 17b of the information processing device 17 to independently function, or are implemented by causing the processor 17a to read a program stored in the memory 17b and execute the program.

As illustrated in Fig. 13, when treatment is interrupted due to a failure or the like of the initial device 3a that is the blood purification device 3 as the information transfer source, the control unit 21 generates response confirmation information and transmits the response confirmation information to the communication unit 18 (A in Fig. 13: response confirmation information). The response confirmation information is transmitted to the mobile terminal 5 via the communication unit 18, and when correspondence by the operator of the mobile terminal 5 is performed, correspondence information is returned to the control unit 21 via the communication unit 18 (B in Fig. 13: correspondence information).

Here, the correspondence information is information as to whether or not the operator can correspond to the processing related to information transfer. For example, information as to whether or not the operator can correspond to information transfer processing is assumed. That is, in a situation where the operator cannot perform the information transfer processing due to another operation, it is necessary to prevent the first and second transfer methods from being selected, and such information is required in order to prevent the transfer method involving intervention of the operator from being selected under a situation where the operator cannot correspond to the information transfer processing.

Next, the selection information generation unit 21d receives, for the patient information, from the storage unit 22, four pieces of transfer method data, installation environment information, and state information which are stored in the storage unit 22 (C in Fig. 13: transfer method data, installation environment information, and state information), and generates control unit selection information based on the correspondence information, the transfer method data, the installation environment information, and the state information. Then, the control unit selection information is final selection information for determining the transfer method. Note that the installation environment information and the state information are appropriately updated periodically or at an arbitrary timing based on update information which is input via the display 13a, the input/output port 13b, or the communication unit 18.

Here, the installation environment information is information related to an environment in which the initial device 3a and the alternative device 3b are set. For example, information as to whether or not a communication line can be used and as to whether there is a reading device or an external storage device using the mobile terminal 5 as an example is assumed. That is, when a communication line cannot be used, it is necessary to prevent the third and fourth transfer methods from being selected, and such information is required in order to prevent the transfer method related to the device that cannot be used in the initial installation state from being selected. In addition, the state information is information related to the use states of the initial device 3a and the alternative device 3b. For example, information related to failures of the display 13a, the input/output port 13b, the communication unit 18, and the reading unit 19 is assumed. In addition, information related to a speed of the communication line and information as to whether or not connection to a server inside and outside the hospital is possible are also assumed. That is, when the display 13a fails, it is necessary to prevent the first transfer method from being selected, and such information is required in order to prevent the transfer method related to the failed device from being selected.

From the above, the installation environment information, the state information, and the correspondence information are information for determining whether or not the first to fourth transfer methods can be executed in the environment where the blood purification device 3 is installed and used.

When the final information transfer method is selected by the selection information generation unit 21d, the control unit 21 receives, from the storage unit 22, the patient information stored in the storage unit 22 (D in Fig. 13: patient information). In addition, the control unit 21 executes transfer processing of the patient information based on the selected information transfer method. Specifically, in a case where the first transfer method using the identification marker is selected, the identification marker generation unit 21b generates an identification marker corresponding to the patient information, transmits the identification marker to the display 13a (E1 in Fig. 13: identification marker), and displays the identification marker on the display 13a. In a case where the second transfer method using the external storage device is selected, the control unit 21 transmits the received patient information to the input/output port 13b (E2 in Fig. 13: patient information), and causes the external storage device connected to the input/output port 13b to store the patient information. In a case where the third transfer method or the fourth transfer method using the in-hospital server 4 or the cloud server 7 is selected, the control unit 21 transmits the received patient information to the server as the transmission destination via the communication unit 18 (E3 in Fig. 13: patient information).

Note that, in a case where the blood purification device 3 is the alternative device 3b as the information transfer destination, since the configuration is the same as the configuration illustrated in Fig. 13 and the flow is the same as the flow of the information illustrated in Fig. 5, the description thereof will be omitted.

Next, processing related to transfer of the patient information in the blood purification system 1 according to the present embodiment will be described with reference to Fig. 14. Note that the processing is different from the processing of the first embodiment up to the selection of the transfer method, and the transfer method itself is the same. Therefore, hereinafter, a case where the first transfer method is selected will be described as an example, and description of a case where another transfer method is selected will be omitted.

First, as illustrated in Fig. 14, treatment is started in one of the blood purification devices 3 (S101). Thereafter, one blood purification device 3 fails due to some reason, and the treatment in the initial device 3a as the blood purification device 3 is interrupted (S102). Note that these steps are the same as S3 and S4 in the first embodiment.

Next, when the control unit 21 of the initial device 3a detects interruption of the treatment, the control unit 21 generates response confirmation information and transmits the response confirmation information to the mobile terminal 5 via the communication unit 18 (T101). For example, the response confirmation information may include question information as to whether or not the operator can correspond to transfer processing of the patient information.

Next, the mobile terminal 5 performs processing of generating correspondence information as to whether or not the operator can correspond to the received response confirmation information. Specifically, in the mobile terminal 5 owned by the operator, when the operator operates the screen displayed on the display 53a, the information processing device 57 of the mobile terminal 5 generates correspondence information that matches the operation. Then, the information processing device 57 of the mobile terminal 5 returns the generated correspondence information to the initial device 3a via the communication unit 58 (T102).

Next, when the control unit 21 of the initial device 3a receives the correspondence information, processing of selecting the transfer method in the initial device 3a is performed (S104). Specifically, the selection information generation unit 21d of the initial device 3a reads, for the patient information stored in the storage unit 22, the four pieces of transfer method data, the installation environment information, and the state information. Then, the selection information generation unit 21d of the initial device 3a generates control unit selection information, which is final selection information for determining the transfer method, based on the received correspondence information and the transfer method data, the installation environment information, and the state information which are read.

Here, an example of a specific method of generating the control unit selection information will be described with reference to Fig. 15. That is, the selection information generation unit 21d performs processing related to the flow of Fig. 15 based on the information and the data which are received or read, and executes selection related to transfer processing of the patient information.

First, the selection information generation unit 21d determines whether or not a communication line can be used in the environment (S151). The determination is performed based on the installation environment information which is read from the storage unit 22.

Next, in the case of Yes in S151, the selection information generation unit 21d determines whether or not the speed of the communication line is equal to or higher than a predetermined value (S152). Subsequently, in the case of Yes in S152, the selection information generation unit 21d determines whether or not connection to the in-hospital server 4 is possible (S153). These determinations are performed based on the state information which is read from the storage unit 22. Then, in the case of Yes in S153, the selection information generation unit 21d generates control unit selection information indicating that the third transfer method is selected (S154).

On the other hand, in the case of No in S153, it is determined whether or not connection to the cloud server 7 that is a server outside the hospital is possible (S155). The determination is performed based on the state information which is read from the storage unit 22. Then, in the case of Yes in S155, the selection information generation unit 21d generates control unit selection information indicating that the fourth transfer method is selected (S156).

In addition, in the case of No in S151 or S152, it is determined whether or not the operator can correspond to transfer processing (S157). The determination is performed based on the received correspondence information. Subsequently, in the case of Yes in S157, the selection information generation unit 21d determines whether or not the display 13a can be used (S158). The determination is performed based on the state information which is read. Then, in the case of Yes in S158, the selection information generation unit 21d generates control unit selection information indicating that the first transfer method is selected (S159).

On the other hand, in the case of No in S158, it is determined whether or not the input/output port 13b can be used (S160). The determination is performed based on the state information which is read from the storage unit 22. Then, in the case of Yes in S160, the selection information generation unit 21d generates control unit selection information indicating that the second transfer method is selected (S161).

In the case of No in S157 or S160, the selection information generation unit 21d determines that the transfer processing of the patient information is impossible because the communication line cannot be used and the operator cannot correspond to the transfer processing, and generates control unit selection information indicating that treatment end is selected (S162). Here, the control unit selection information indicating that the treatment is ended is displayed as suggested information or recommended information on the display 13a of the initial device 3a.

Note that the above flow is merely an example, and the order of each determination may be changed. That is, the order of each determination can be appropriately set such that any one of the first to fourth transfer methods is preferentially selected. Further, the selection information generation unit 21d may include an AI determination unit or the like that is obtained by performing machine learning based on the response information, the four pieces of transfer method data, the installation environment information, and the state information without performing determination as illustrated in Fig. 15. Thereby, the selection information generation unit 21d may extract an optimal method corresponding to a situation when information transfer is performed.

Returning to Fig. 14, in the initial device 3a, processing of displaying the identification marker is performed similarly to the processing of S6 in the first embodiment (S105). Subsequently, in the mobile terminal 5, similarly to the processing of S7 to S9 in the first embodiment, imaging processing (S106), patient information display processing (S107), and identification marker display processing (S108) are performed. Thereafter, in the alternative device 3b, similarly to the processing of S10 to S15 in the first embodiment, the imaging processing (S109), the patient information processing (S110), the processing of displaying the transfer procedure information (S111), the processing of displaying the difference information (S112), the transfer processing (S113), and the treatment restart processing (S114) are performed.

Note that, in the present embodiment, the response confirmation is performed on the mobile terminal 5, but selection of the transfer method may be performed without performing the response confirmation. That is, when selection of the transfer method is performed, the transfer method may be selected based on only the remaining information without considering the information as to whether or not the operator can correspond to the transfer processing.

### (Operation and Effect of Second Embodiment)

Also in the present embodiment, four types of transfer methods for transferring the patient information are stored in the information processing device 17 of the blood purification device 3, and the transfer method is automatically selected by the information processing device 17 at a stage immediately before the information transfer. That is, according to the environments or the like of the blood purification device 3 and the blood purification system 1, any one of the transfer methods in which the used devices are different is automatically selected, and the information transfer is automatically performed.

Therefore, an optimal transfer method corresponding to the states of the blood purification device 3 and the blood purification system 1 or the installation environments of the blood purification device 3 and the blood purification system 1 can be automatically selected, and it is possible to easily and accurately perform the information transfer and shorten a lead time until the treatment is restarted. In particular, in the present embodiment, since the information processing device 17 automatically selects the transfer method and the information transfer automatically proceeds, the information transfer can be quickly and accurately performed. In addition, since it is not necessary to input, to the blood purification device 3, various types of information related to the patient and the transfer of the blood purification device 3, a human error such as an input error does not occur, and the overall processing time can be reduced.

### <Third Embodiment>

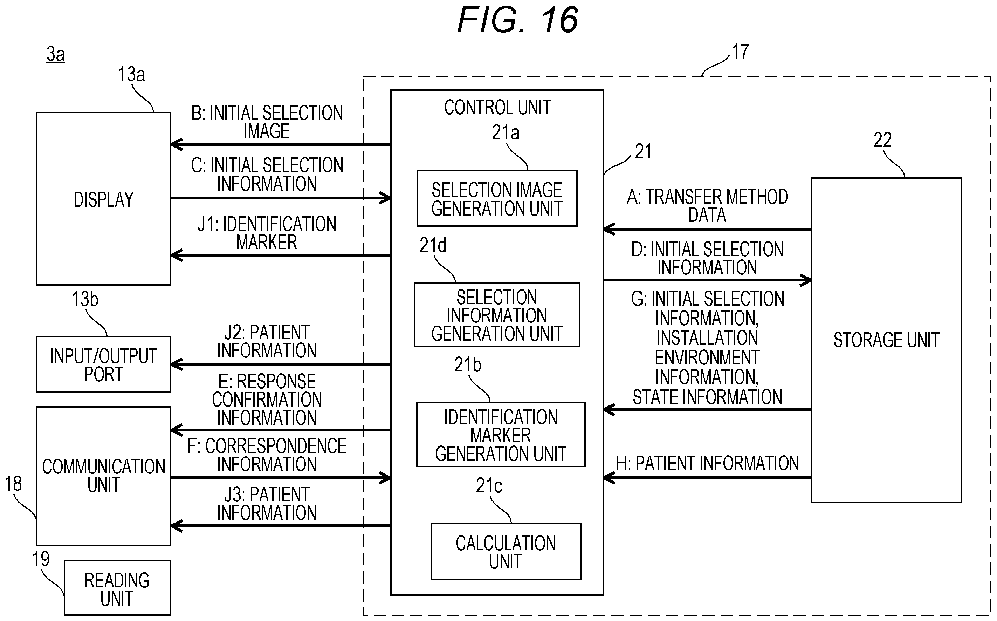

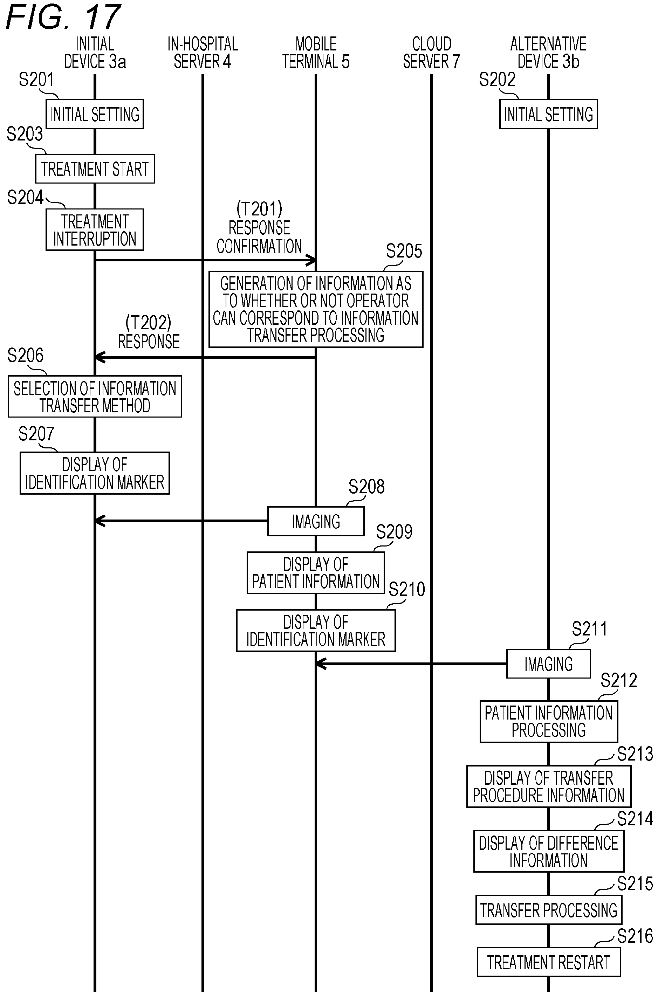

In the first embodiment and the second embodiment, the method of transferring the patient information is determined by selection by either the operator of the blood purification device 3 or the control unit 21 of the blood purification device 3. However, in an initial stage, the selection may be performed by the operator, and the control unit 21 may perform final selection immediately before the information transfer. That is, the initial selection information including at least one of the first to fourth transfer methods may be generated by the selection of the operator, and the control unit 21 may generate the control unit selection information that is the final selection information based on the initial selection information, the installation environment information of the blood purification device 3, the state information of the blood purification device 3, and the correspondence information of the operator. Such a case will be described as a third embodiment with reference to Fig. 16 and Fig. 17. Here, Fig. 16 is a functional block diagram related to information transfer of the blood purification device 3 according to the third embodiment. Fig. 17 is a sequence diagram related to information transfer in the blood purification system 1 according to the third embodiment. Note that parts different from the first and second embodiments will be basically described, description of the same content will be omitted, and the same reference numerals will be basically used in the drawings.

First, as illustrated in Fig. 16, the control unit 21 of the information processing device 17 according to the present embodiment includes a selection image generation unit 21a, an identification marker generation unit 21b, a calculation unit 21c, and a selection information generation unit 21d. That is, unlike the first and second embodiments, the control unit 21 includes both the selection image generation unit 21a that generates an image to be selected by the operator and the selection information generation unit 21d that generates selection information for selecting a transfer method. Similarly to the first and second embodiments, these units are implemented by causing the processor 17a and the memory 17b of the information processing device 17 to independently function, or are implemented by causing the processor 17a to read a program stored in the memory 17b and execute the program.

As illustrated in Fig. 16, in a case where the blood purification device 3 is the initial device 3a as the information transfer source, the selection image generation unit 21a receives four pieces of transfer method data of the patient information stored in the storage unit 22 from the storage unit 22 (A in Fig. 16: transfer method data), and generates an initial selection image for allowing the operator to select the transfer method in an initial stage. In addition, the selection image generation unit 21a transmits the generated initial selection image to the display 13a (B in Fig. 16: initial selection image). Further, the selection image generation unit 21a receives, from the display 13a, initial selection information that is input by the operator of the blood purification device 3 operating the display 13a (C in Fig. 16: initial selection information). Then, the selection image generation unit 21a transmits the received initial selection information to the storage unit 22 (D in Fig. 16: initial selection information), and the initial selection information is stored in the storage unit 22.

Thereafter, when treatment is interrupted due to a failure or the like of the initial device 3a that is the blood purification device 3 as the information transfer source, the control unit 21 generates response confirmation information and transmits the response confirmation information to the communication unit 18 (E in Fig. 16: response confirmation information). The response confirmation information is transmitted to the mobile terminal 5 via the communication unit 18, and when correspondence by the operator of the mobile terminal 5 is performed, correspondence information is returned to the control unit 21 via the communication unit 18 (F in Fig. 16: correspondence information).

Next, the selection information generation unit 21d receives, from the storage unit 22, the initial selection information, the installation environment information, and the state information which are stored in the storage unit 22 (G in Fig. 16: initial selection information, installation environment information, and state information), and generates control unit selection information based on the correspondence information, the initial selection information, the installation environment information, and the state information. Then, the control unit selection information is final selection information for determining the transfer method.

When the final information transfer method is selected by the selection information generation unit 21d, the control unit 21 receives, from the storage unit 22, the patient information stored in the storage unit 22 (H in Fig. 16: patient information). In addition, the control unit 21 executes transfer processing of the patient information based on the selected information transfer method (in Fig. 16, J1: identification marker, J2: patient information, J3: patient information).

Note that, in a case where the blood purification device 3 is the alternative device 3b as the information transfer destination, since the configuration is the same as the configuration illustrated in Fig. 16 and the flow is the same as the flow of the information illustrated in Fig. 5, the description thereof will be omitted.

Next, processing related to transfer of the patient information in the blood purification system 1 according to the present embodiment will be described with reference to Fig. 17. Note that the processing is different from the processing of the first and second embodiments up to the selection of the transfer method, and the transfer method itself is the same. Therefore, hereinafter, a case where the first transfer method is selected will be described as an example, and description of a case where another transfer method is selected will be omitted.

First, similarly to the initial setting (S1, S2), the treatment start (S3), and the treatment interruption (S4) in the first embodiment, in the initial device 3a and the alternative device 3b, initial setting (S201, S202), treatment start (S203), and treatment interruption (S204) are performed.

Next, similarly to the response confirmation (T101) in the second embodiment, the control unit 21 generates response confirmation information and transmits the response confirmation information to the mobile terminal 5 via the communication unit 18 (T201). Subsequently, similarly to the generation (S103) and the returning (T102) of the correspondence information in the second embodiment, the mobile terminal 5 performs processing of generating and returning correspondence information as to whether the operator can correspond to the received response confirmation information (S205, T202).

Next, when the control unit 21 of the initial device 3a receives the correspondence information, processing of selecting the transfer method in the initial device 3a is performed (S206). Specifically, the selection information generation unit 21d of the initial device 3a reads the initial selection information, the installation environment information, and the state information which are stored in the storage unit 22. Then, the selection information generation unit 21d of the initial device 3a generates control unit selection information, which is final selection information for determining the transfer method, based on the received correspondence information and the initial selection information, the installation environment information, and the state information which are read.

The selection processing can be executed by a determination flow modified so as to prioritize the initially-selected transfer method based on the flow illustrated in Fig. 15 of the second embodiment. In addition, the selection information generation unit 21d may include an AI determination unit or the like that is obtained by performing machine learning based on the response information, the four pieces of transfer method data, the installation environment information, the state information, and the initial selection information without performing determination by the determination flow. Thereby, the selection information generation unit 21d may extract an optimal method corresponding to a situation when information transfer is performed.

In the initial device 3a, identification marker display processing is performed similarly to the processing of S6 in the first embodiment (S207). Subsequently, in the mobile terminal 5, similarly to the processing of S7 to S9 in the first embodiment, imaging processing (S208), patient information display processing (S209), and identification marker display processing (S210) are performed. Thereafter, in the alternative device 3b, similarly to the processing of S10 to S15 in the first embodiment, the imaging processing (S211), the patient information processing (S212), the processing of displaying the transfer procedure information (S213), the processing of displaying the difference information (S214), the transfer processing (S215), and the treatment restart processing (S216) are performed.

### (Operation and Effect of Third Embodiment)

In the present embodiment, in a state where four types of transfer methods for transferring the patient information are stored in the information processing device 17 of the blood purification device 3, the operator selects the transfer method at an initial stage, and the information processing device 17 automatically determines the final transfer method immediately before the information transfer. That is, according to the environments or the like of the blood purification device 3 and the blood purification system 1, any one of the transfer methods in which the used devices are different is selected and information transfer is performed.

Therefore, it is possible to adopt an optimal transfer method corresponding to the states of the blood purification device 3 and the blood purification system 1 or the installation environments of the blood purification device 3 and the blood purification system 1, and it is possible to easily and accurately perform information transfer and shorten a lead time until the treatment is restarted. In particular, in the present embodiment, the operator can select a transfer method at an initial stage, and select a plurality of methods adapted to the environments of the blood purification device 3 and the blood purification system 1. Further, the information processing device 17 automatically selects the final transfer method, and the information transfer automatically proceeds. Thus, the information transfer can be quickly and accurately performed while reflecting the site situation of the hospital 2. In addition, since it is not necessary to input, to the blood purification device 3, various types of information related to the patient and the transfer of the blood purification device 3, a human error such as an input error does not occur, and the overall processing time can be reduced.

### <Fourth Embodiment>

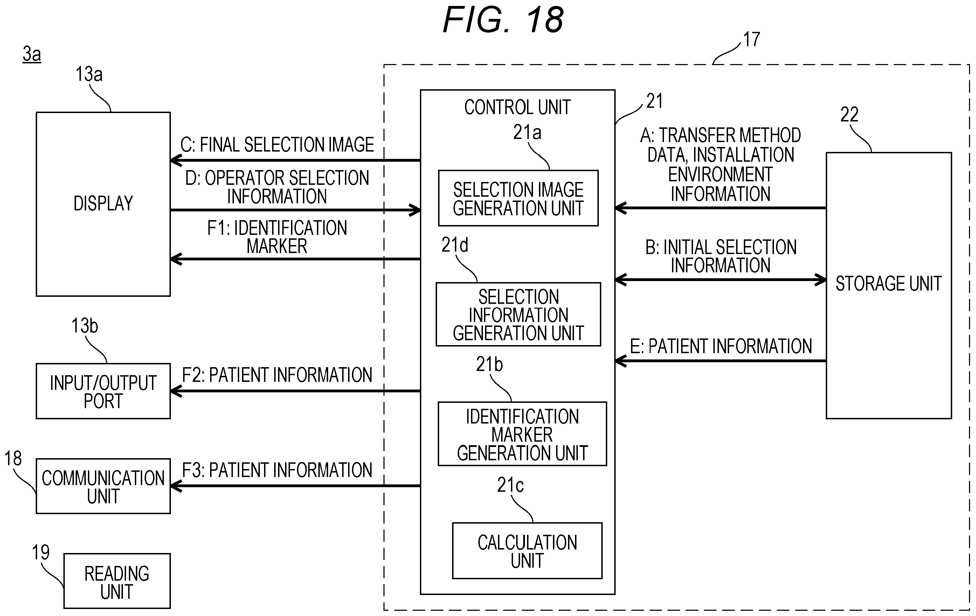

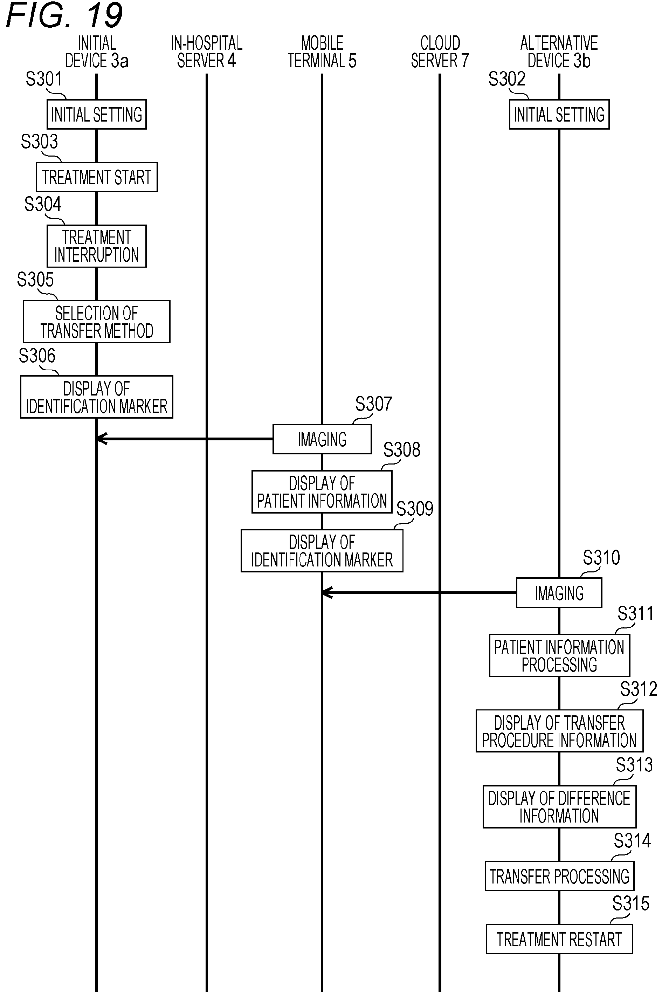

In the third embodiment, the selection in the initial stage is performed by the operator, and the final selection is performed by the control unit 21, but this may be reversed. That is, the selection of the initial stage may be performed by the control unit 21, the recommended transfer method may be exemplified, and the final selection may be performed by the operator. Such a case will be described as a fourth embodiment with reference to Fig. 18 and Fig. 19. Here, Fig. 18 is a functional block diagram related to information transfer of the blood purification device 3 (the initial device 3a) according to the fourth embodiment. Fig. 19 is a sequence diagram related to information transfer in the blood purification system 1 according to the fourth embodiment. Note that parts different from the first to third embodiments will be basically described, description of the same content will be omitted, and the same reference numerals will be basically used in the drawings.

First, as illustrated in Fig. 18, the control unit 21 of the information processing device 17 according to the present embodiment includes a selection image generation unit 21a, an identification marker generation unit 21b, a calculation unit 21c, and a selection information generation unit 21d. That is, unlike the first and second embodiments, the control unit 21 includes both the selection image generation unit 21a that generates an image to be selected by the operator and the selection information generation unit 21d that generates selection information for selecting a transfer method. Similarly to the first and second embodiments, these units are implemented by causing the processor 17a and the memory 17b of the information processing device 17 to independently function, or are implemented by causing the processor 17a to read a program stored in the memory 17b and execute the program.

As illustrated in Fig. 18, in a case where the blood purification device 3 is the initial device 3a as the information transfer source, the selection information generation unit 21d receives, for the patient information, from the storage unit 22, the four pieces of transfer method data and the installation environment information which are stored in the storage unit 22 (A of Fig. 18: transfer method data, installation environment information), and generates control unit selection information based on the received data and information. Then, the control unit selection information is initial selection information for determining the transfer method.

Thereafter, when treatment is interrupted due to a failure or the like of the initial device 3a which is the blood purification device 3, the selection image generation unit 21a receives, from the storage unit 22, the initial selection information stored in the storage unit 22 (B in Fig. 18: initial selection information), and generates a final selection image for allowing the operator to select the transfer method at a stage of actually performing the information transfer. In addition, the selection image generation unit 21a transmits the generated final selection image to the display 13a (C in Fig. 18: final selection image). Further, the selection image generation unit 21a receives, from the display 13a, operator selection information that is input by the operator of the blood purification device 3 operating the display 13a (D in Fig. 18: operator selection information).

When the final method of the information transfer is selected, the control unit 21 receives, from the storage unit 22, the patient information stored in the storage unit 22 (E in Fig. 18: patient information). In addition, the control unit 21 executes transfer processing of the patient information based on the selected information transfer method (in Fig. 18, F1: identification marker, F2: patient information, F3: patient information).

Note that, in a case where the blood purification device 3 is the alternative device 3b as the information transfer destination, since the configuration is the same as the configuration illustrated in Fig. 18 and the flow is the same as the flow of the information illustrated in Fig. 5, the description thereof will be omitted.

Next, processing related to transfer of the patient information in the blood purification system 1 according to the present embodiment will be described with reference to Fig. 19. Note that the processing is different from the processing of the first to third embodiments up to the selection of the transfer method, and the transfer method itself is the same. Therefore, hereinafter, a case where the first transfer method is selected will be described as an example, and description of a case where another transfer method is selected will be omitted.

First, as illustrated in Fig. 19, in each of the blood purification devices 3 (the initial device 3a and the alternative device 3b), initial setting related to information transfer is performed (S301 and S302). The selection information generation unit 21d of the initial device 3a and the alternative device 3b reads the transfer method data and the installation environment information which are stored in the storage unit 22. Then, the selection information generation unit 21d generates control unit selection information that is the initial selection information for determining the transfer method based on the data and the information.

The selection processing can be executed by a determination flow, which is modified to delete the determination based on the state information and the response information, based on the flow illustrated in Fig. 15 of the second embodiment. In this case, instead of selecting only one transfer method, a plurality of transfer methods may be selected. In addition, the selection information generation unit 21d may include an AI determination unit or the like that is obtained by performing machine learning based on the four pieces of transfer method data and the installation environment information without performing the determination by the determination flow. Thus, the selection information generation unit 21d may extract at least one optimal method corresponding to the situation when performing the information transfer.

Next, similarly to S3 to S6 of the first embodiment, in the initial device 3a, treatment start (S303), treatment interruption (S304), selection of the transfer method (S305), and display of the identification marker (S306) are performed. Subsequently, in the mobile terminal 5, similarly to the processing of S7 to S9 in the first embodiment, imaging processing (S307), patient information display processing (S308), and identification marker display processing (S309) are performed. Thereafter, in the alternative device 3b, similarly to the processing of S10 to S15 in the first embodiment, the imaging processing (S310), the patient information processing (S311), the processing of displaying the transfer procedure information (S312), the processing of displaying the difference information (S313), the transfer processing (S314), and the treatment restart processing (S315) are performed.

### (Operation and Effect of Fourth Embodiment)

In the present embodiment, in a state where four types of transfer methods for transferring the patient information are stored in the information processing device 17 of the blood purification device 3, the information processing device 17 automatically selects the transfer method at an initial stage, and the operator finally determines the transfer method immediately before the information transfer. That is, according to the environments or the like of the blood purification device 3 and the blood purification system 1, any one of the transfer methods in which the used devices are different is selected and information transfer is performed.

Therefore, it is possible to adopt an optimal transfer method corresponding to the states of the blood purification device 3 and the blood purification system 1 or the installation environments of the blood purification device 3 and the blood purification system 1, and it is possible to easily and accurately perform information transfer and shorten a lead time until the treatment is restarted. In particular, in the present embodiment, since the information processing device 17 automatically selects the transfer method at the initial stage, the selection of the information transfer by the operator immediately before the information transfer can be performed more easily and quickly, and the site situation of the hospital 2 can be reflected. In addition, since it is not necessary to input, to the blood purification device 3, various types of information related to the patient and the transfer of the blood purification device 3, a human error such as an input error does not occur, and the overall processing time can be reduced.

### <Embodiments of Present Disclosure>

According to a first aspect of the present disclosure, there is provided a blood purification device that purifies blood of a patient, the blood purification device including: a storage unit that stores patient information including patient-specific data and treatment data; an output unit that outputs the patient information to the outside via an information acquisition device used by an operator of the blood purification device; a communication unit that transmits the patient information to the outside using a communication line; and a control unit that performs transfer of the patient information by controlling either the output unit or the communication unit.

As described above, since any one of the transfer methods in which the used devices are different is selected and the information transfer is performed, an optimal transfer method corresponding to the state of the blood purification device or the installation environment of the blood purification device can be adopted. Thus, the information transfer can be easily and accurately performed, and the lead time until the treatment is restarted can be shortened.

According to a second aspect of the present disclosure, in the first aspect, the output unit includes a display that displays the patient information as an identification marker and an output port to which the information acquisition device is connected, and outputs the patient information via either the display or the output bort, and the communication unit transmits the patient information to an in-facility server provided in a facility in which the blood purification device is installed or an out-of-facility server provided outside the facility via the communication line. Thereby, it is possible to perform the information transfer in consideration of the used device in more detail, and thus, it is possible to shorten the lead time until the treatment is restarted while more easily and accurately performing the information transfer.

According to a third aspect of the present disclosure, in the second aspect, the information acquisition device is a reading device that reads the identification marker or an external storage device that is connected to the output port, and the control unit determines, based on final selection information, any one of a first transfer method, a second transfer method, a third transfer method, and a fourth transfer method, the first transfer method being a method of generating the identification marker, displaying the identification marker on the display, and performing information transfer by reading performed of the reading device, the second transfer method being a method of performing information transfer by storing the patient information in the external storage device via the output unit, the third transfer method being a method of performing information transfer by transmitting the patient information to the in-facility server via the communication unit, and the fourth transfer method being a method of performing information transfer by transmitting the patient information to the out-of-facility server via the communication unit. Thereby, it is possible to perform the information transfer in consideration of the used device in more detail, and thus, it is possible to shorten the lead time until the treatment is restarted while more easily and accurately performing the information transfer.

According to a fourth aspect of the present disclosure, in the third aspect, the final selection information is either operator selection information selected by the operator or control unit selection information selected by the control unit. Thereby, since the transfer method is finally selected by a person who understands the environment of the blood purification device or the blood purification device, the information transfer can be easily and quickly performed.

According to a fifth aspect of the present disclosure, in the fourth aspect, the storage unit stores initial selection information including at least one of the first to fourth transfer methods that is initially selected by the operator, and
before transfer of the patient information, the control unit displays a final selection image of the transfer method on the display based on the initial selection information, and acquires the operator selection information. Thereby, since the transfer method is selected in stages by the operator who understands the environment of the blood purification device, it is possible to easily and quickly perform the optimal information transfer.

According to a sixth aspect of the present disclosure, in the fourth aspect, the storage unit stores initial selection information including at least one of the first to fourth transfer methods that is initially selected by the operator, and
the control unit generates the control unit selection information based on the initial selection information, installation environment information of the blood purification device, state information of the blood purification device, and correspondence information of the operator. The selection at an initial stage is performed by a person who understands the environment of the blood purification device, and the transfer method is automatically selected by the device immediately before the information transfer. Thus, the information transfer can be easily and quickly performed.

According to a seventh aspect of the present disclosure, in the fourth aspect, the control unit generates initial selection information including at least one of the first to fourth transfer methods based on installation environment information of the blood purification device, and stores the initial selection information in the storage unit, and
before transfer of the patient information, the control unit displays a final selection image of the transfer method on the display based on the initial selection information, and acquires the operator selection information. Thereby, since the transfer method is automatically selected by the device that recognizes the environment of the blood purification device, the information transfer can be easily and quickly performed.

According to an eighth aspect of the present disclosure, in any one of the second to seventh aspects, the control unit displays other patient information transferred from another blood purification device on the display. Thereby, it is possible to easily recognize the patient information to be transferred, and thus, it is possible to easily and quickly perform the information transfer.

According to a ninth aspect of the present disclosure, in any one of the second to eighth aspects, the control unit generates transfer procedure information for resuming treatment of the patient based on the other patient information, and displays the transfer procedure information on the display. Thereby, it is possible to easily recognize the transfer procedure, and thus, it is possible to easily and quickly perform the information transfer.

According to a tenth aspect of the present disclosure, in any one of the second to ninth aspects, the control unit extracts difference information by comparing the patient information stored in the storage unit with the other patient information transferred from the other blood purification device, and displays the difference information on the display. Thereby, in the processing related to the information transfer, it is possible to easily recognize the content different from the previous content, and thus, it is possible to easily and quickly perform the information transfer.

According to an eleventh aspect of the present disclosure, in any one of the second to tenth aspects, the blood purification device further includes: a reading unit that reads the identification marker displayed as the other patient information; and an input port to which the information acquisition device storing the other patient information is connected and to which the other patient information is input. Thereby, since the patient information can be transferred using the two blood purification devices, the information transfer can be easily and quickly performed.

According to a twelfth aspect of the present disclosure, there is provided a blood purification system including: a first blood purification device that purifies blood of a patient; a second blood purification device that is selected as an alternative device for the first blood purification device; and a server that is connected to the first blood purification device and the second blood purification device via a communication line, in which the first blood purification device includes a storage unit that stores patient information including patient-specific data and treatment data, an output unit that outputs the patient information to the outside via an information acquisition device used by an operator of the first blood purification device, a communication unit that transmits the patient information to the server using the communication line, and a control unit that performs processing for transferring the patient information to the second blood purification device by controlling either the output unit or the communication unit. As described above, since either the transfer methods in which the used devices are different is selected and the information transfer is performed, an optimal transfer method corresponding to the state of the blood purification device or the installation environment of the blood purification device can be adopted. Thus, the information transfer can be easily and accurately performed, and the lead time until the treatment is restarted can be shortened.

According to a thirteenth aspect of the present disclosure, in the twelfth aspect, the server generates transfer procedure information for resuming treatment of the patient in the second blood purification device and difference information between the first blood purification device and the second blood purification device based on the patient information received from the first blood purification device and past patient information of the second blood purification device, and transmits the transfer procedure information and the difference information to the second blood purification device.

Thereby, it is possible to easily recognize the patient information to be transferred, the transfer procedure, and the content different from the previous content that is related to the information transfer. Thus, the information transfer can be easily and quickly performed.

According to a fourteenth aspect of the present disclosure, there is provided an information transfer method of transferring patient information from a blood purification device that purifies blood of a patient, the information transfer method including: a step of storing the patient information including patient-specific data and treatment data in a storage unit of the blood purification device; a step of selecting either an operator-intervention-type transfer method or a communication-type transfer method, the operator-intervention-type transfer method being a method of transferring information by outputting the patient information to the outside via an information acquisition device used by an operator of the blood purification device, and the communication-type transfer method being a method of transferring information by transmitting the patient information to the outside using a communication line; and a step of transferring the patient information based on the selected transfer method. As described above, since the information transfer is performed by selecting either an operator-intervention-type transfer method and a communication-type transfer method in which the used devices are different, it is possible to adopt an optimal transfer method corresponding to the state of the blood purification device or the installation environment of the blood purification device. Thus, it is possible to easily and accurately perform information transfer, and to shorten the lead time until treatment is restarted.

### Reference Signs List

- 1: Blood purification system
- 2: Hospital
- 3: Blood purification device
- 4: In-hospital server (in-facility server)
- 5: Mobile terminal
- 7: Cloud server (out-of-facility server)
- 13: Input/output unit
- 13a: Display
- 13b: Input/output port
- 17: Information processing device
- 18: Communication unit
- 19: Reading unit
- 21: Control unit
- 22: Storage unit

## Claims

1. A blood purification device that purifies blood of a patient, the blood purification device comprising:
a storage unit that stores patient information including patient-specific data and treatment data;
an output unit that outputs the patient information to the outside via an information acquisition device used by an operator of the blood purification device;
a communication unit that transmits the patient information to the outside using a communication line; and
a control unit that performs transfer of the patient information by controlling either the output unit or the communication unit.

2. The blood purification device according to claim 1, wherein
the output unit includes a display that displays the patient information as an identification marker and an output port to which the information acquisition device is connected, and outputs the patient information via either the display or the output bort, and
the communication unit transmits the patient information to an in-facility server provided in a facility in which the blood purification device is installed or an out-of-facility server provided outside the facility via the communication line.

3. The blood purification device according to claim 2, wherein
the information acquisition device is a reading device that reads the identification marker or an external storage device that is connected to the output port, and
the control unit determines, based on final selection information, any one of a first transfer method, a second transfer method, a third transfer method, or a fourth transfer method,
the first transfer method being a method of generating the identification marker, displaying the identification marker on the display, and performing information transfer by reading performed of the reading device,
the second transfer method being a method of performing information transfer by storing the patient information in the external storage device via the output unit,
the third transfer method being a method of performing information transfer by transmitting the patient information to the in-facility server via the communication unit, and
the fourth transfer method being a method of performing information transfer by transmitting the patient information to the out-of-facility server via the communication unit.

4. The blood purification device according to claim 3, wherein
the final selection information is either operator selection information selected by the operator or control unit selection information selected by the control unit.

5. The blood purification device according to claim 4, wherein
the storage unit stores initial selection information including at least one of the first to fourth transfer methods that is initially selected by the operator, and
before transfer of the patient information, the control unit displays a final selection image of the transfer method on the display based on the initial selection information, and acquires the operator selection information.

6. The blood purification device according to claim 4, wherein
the storage unit stores initial selection information including at least one of the first to fourth transfer methods that is initially selected by the operator, and
the control unit generates the control unit selection information based on the initial selection information, installation environment information of the blood purification device, state information of the blood purification device, and correspondence information of the operator.

7. The blood purification device according to claim 4, wherein
the control unit generates initial selection information including at least one of the first to fourth transfer methods based on installation environment information of the blood purification device, and stores the initial selection information in the storage unit, and
before transfer of the patient information, the control unit displays a final selection image of the transfer method on the display based on the initial selection information, and acquires the operator selection information.

8. The blood purification device according to claim 2, wherein
the control unit displays other patient information transferred from another blood purification device on the display.

9. The blood purification device according to claim 8, wherein
the control unit generates transfer procedure information for restarting treatment of the patient based on the other patient information, and displays the transfer procedure information on the display.

10. The blood purification device according to claim 8 or 9, wherein
the control unit extracts difference information by comparing the patient information stored in the storage unit with the other patient information transferred from the other blood purification device, and displays the difference information on the display.

11. The blood purification device according to claim 8, further comprising:
a reading unit that reads the identification marker displayed as the other patient information; and
an input port to which the information acquisition device storing the other patient information is connected and to which the other patient information is input.

12. A blood purification system comprising:
a first blood purification device that purifies blood of a patient;
a second blood purification device that is selected as an alternative device for the first blood purification device; and
a server that is connected to the first blood purification device and the second blood purification device via a communication line, wherein
the first blood purification device includes
a storage unit that stores patient information including patient-specific data and treatment data,
an output unit that outputs the patient information to the outside via an information acquisition device used by an operator of the first blood purification device,
a communication unit that transmits the patient information to the server using the communication line, and
a control unit that performs processing for transferring the patient information to the second blood purification device by controlling either the output unit or the communication unit.

13. The blood purification system according to claim 12, wherein
the server generates transfer procedure information for restarting treatment of the patient in the second blood purification device and difference information between the first blood purification device and the second blood purification device based on the patient information received from the first blood purification device and past patient information of the second blood purification device, and transmits the transfer procedure information and the difference information to the second blood purification device.

14. An information transfer method of transferring patient information from a blood purification device that purifies blood of a patient, the information transfer method comprising:
a step of storing the patient information including patient-specific data and treatment data in a storage unit of the blood purification device;
a step of selecting either an operator-intervention-type transfer method or a communication-type transfer method, the operator-intervention-type transfer method being a method of transferring information by outputting the patient information to the outside via an information acquisition device used by an operator of the blood purification device, and the communication-type transfer method being a method of transferring information by transmitting the patient information to the outside using a communication line; and
a step of transferring the patient information based on the selected transfer method.
